# EUROPEAN PATENT APPLICATION

(11) **EP 4 851 178 A1**
(43) Date of publication of application: **09.12.2026**
(21) Application number: 25748665.4
(22) Date of filing: 29.01.2025
(51) Int. Cl.: C07K 2/00, C07K 1/02, C07K 14/435

(54) **BLOCK COPOLYMER, METHOD FOR PRODUCING BLOCK COPOLYMER, AND ARTIFICIAL PROTEIN**

(30) Priority: 29.01.2024 JP 2024010946
(71) Applicant: Spiber Inc., Tsuruoka-shi, Yamagata 997-0052 (JP)
(72) Inventor: MUTO Fumiya, Tsuruoka-shi, Yamagata 997-0052 (JP); SUGAWARA Hiroaki, Tsuruoka-shi, Yamagata 997-0052 (JP); HAUSER Jacob Richard, Tsuruoka-shi, Yamagata 997-0052 (JP)
(74) Representative: TBK
(86) International application number: PCT/JP2025/002764
(87) International publication number: WO 2025/164661

(57) **Abstract**

A block copolymer and the like having a first segment including a protein structure, and one or a plurality of second segments including a molecular group having a function of plasticizing the protein structure and bound to the first segment, in which the protein structure has a main sequence and a hydrophobic tag bound to an N-terminus and a C-terminus of the main sequence, and has at least two reaction sites that react with a compound having the second segment and a reactive group, among the reaction sites, the reaction site closest to the N-terminus side is present within the hydrophobic tag on the N-terminus side, is NH₂ that is the N-terminus of the protein structure, or is present within the main sequence, and among the reaction sites, the reaction site closest to the C-terminus side is present within the hydrophobic tag on the C-terminus side or is present within 9 residues from the C-terminus of the main sequence.

## Description

### Technical Field

The present invention relates to a block copolymer, a method for producing a block copolymer, and an artificial protein.

### Background Art

Currently, in various fields, proteins or block copolymers in which a protein is bound to another structure are industrially used.

For example, a function such as flexibility is added as a block copolymer by binding a molecule having a plasticizing function to a protein.

For example, Patent Literature 1 describes a synthetic polymer having a first segment including a polypeptide backbone and one or a plurality of second segments bound to the first segment, in which the second segment includes a molecular group having a plasticizing function with respect to the polypeptide backbone.

### Citation List

### Patent Literature

Patent Literature 1: WO 2021/187502 A

### Summary of Invention

### Technical Problem

In recent years, block copolymer technology has attracted great attention for highly versatile applications in various industries such as film production, leather processing, and fiber production.

At the same time, the utilization of structural proteins such as fibroin as an alternative to conventional structural materials has emerged as a notable trend, mainly due to their excellent durability and robustness. Furthermore, as described in Patent Literature 1, there is an increasing interest in developing a block copolymer material containing fibroin and improving the overall properties thereof in order to cope with the unique advantages of fibroin.

However, in the production of a block copolymer material composed of fibroin, there is a problem that productivity is low and reactivity between fibroin and other polymer components essential for block copolymer synthesis is low despite an increasing demand for the block copolymer material. Furthermore, the manufacturing process itself is complex, and it is difficult to realize effective mass production.

Addressing these problems is critical to enabling efficient mass production and fully realizing the potential of these advanced materials.

The present inventors have focused on these problems, studied to improve a process for producing a block copolymer containing fibroin, and completed the present invention.

An object of the present invention is to provide a block copolymer having excellent productivity, a method for producing the same, and an artificial protein having excellent productivity of the block copolymer.

### Solution to Problem

Representative aspects of the present invention are shown below.
<1> A block copolymer having a first segment including a protein structure, and one or a plurality of second segments including a molecular group having a function of plasticizing the protein structure and bound to the first segment, in which
   the protein structure has a main sequence and a hydrophobic tag bound to an N-terminus and a C-terminus of the main sequence, and has at least two reaction sites that react with a compound having the second segment and a reactive group,
   among the reaction sites, the reaction site closest to the N-terminus side is present within the hydrophobic tag on the N-terminus side, is NH₂ that is the N-terminus of the protein structure, or is present within the main sequence, and
   among the reaction sites, the reaction site closest to the C-terminus side is present within the hydrophobic tag on the C-terminus side or is present within 9 residues from the C-terminus of the main sequence.
<2> The block copolymer according to <1>, in which the reaction site closest to the C-terminus side among the reaction sites is present at the C-terminus of the hydrophobic tag on the C-terminus side, or is present within 9 residues from the C-terminus of the main sequence.
<3> The block copolymer according to <1> or <2>, in which the reaction site closest to the N-terminus side among the reaction sites is present at the N-terminus of the hydrophobic tag on the N-terminus side, or is present within the main sequence.
<4> The block copolymer according to any one of <1> to <3>, in which, among the reaction sites, the reaction site closest to the N-terminus side, which is present within the main sequence, and the reaction site closest to the C-terminus side, which is present within the hydrophobic tag on the C-terminus side or is present within 9 residues from the C-terminus of the main sequence, are thiol groups.
<5> The block copolymer according to <4>, in which the compound has a thiol-reactive group that reacts with the thiol.
<6> The block copolymer according to any one of <1> to <3>, in which, among the reaction sites, the reaction site closest to the N-terminus side, which is present within the hydrophobic tag on the N-terminus side, and the reaction site closest to the C-terminus side, which is present within the hydrophobic tag on the C-terminus side or is present within 9 residues from the C-terminus of the main sequence, are amino groups.
<7> The block copolymer according to <6>, in which the compound has an amino-reactive group that reacts with the amine.
<8> The block copolymer according to any one of <1> to <7>, in which the hydrophobic tag is GFIL or two or more repeats of GFIL.
<9> The block copolymer according to any one of <1> to <8>, in which a molecular weight of the protein structure is 1 to 300 kDa.
<10> A method for producing a block copolymer, the method including binding a compound having a first segment including a protein structure, and a compound having a second segment including a molecular group having a function of plasticizing the protein structure, in which
   the protein structure has a main sequence and a hydrophobic tag bound to an N-terminus and a C-terminus of the main sequence, and has at least two reaction sites that react with a compound having the second segment and a reactive group,
   among the reaction sites, the reaction site closest to the N-terminus side is present within the hydrophobic tag on the N-terminus side, is NH₂ that is the N-terminus of the protein structure, or is present within the main sequence, and
   among the reaction sites, the reaction site closest to the C-terminus side is present within the hydrophobic tag on the C-terminus side or is present within 9 residues from the C-terminus of the main sequence.
<11> The method for producing a block copolymer according to <10>, including a step of binding the compound having the first segment and the compound having the second segment by a mechanochemical treatment.
<12> The method for producing a block copolymer according to <10>, including adding the compound having the first segment and the compound having the second segment, to a solvent, in which the compound having the second segment is added in a non-solution state.
<13> The method for producing a block copolymer according to <12>, including adding a reducing agent, a compound having the first segment, and a compound having the second segment, to the solvent in this order.
<14> An artificial protein having a main sequence and a hydrophobic tag bound to an N-terminus and a C-terminus of the main sequence, and the artificial protein including hydrophobic tags at the N-terminus and the C-terminus, in which
   the artificial protein contains at least two cysteine residues,
   among the cysteine residues, a cysteine residue closest to the N-terminus side is present within the main sequence, and
   among the cysteine residues, a cysteine residue closest to the C-terminus side is present within the hydrophobic tag on the C-terminus side or present within 9 residues from the C-terminus of the main sequence.
<15> An artificial protein having a main sequence and a hydrophobic tag bound to an N-terminus and a C-terminus of the main sequence, and the artificial protein including hydrophobic tags at the N-terminus and the C-terminus, in which
   the artificial protein contains at least one lysine residue,
   among the lysine residues, a lysine residue closest to the N-terminus side is present within the hydrophobic tag on the N-terminus side, or no lysine residue is present within the hydrophobic tag on the N-terminus side, and
   among the lysine residues, a lysine residue closest to the C-terminus side is present within the hydrophobic tag on the C-terminus side or present within 9 residues from the C-terminus of the main sequence.
<16> The artificial protein according to <14> or <15>, in which the hydrophobic tag is GFIL or two or more repeats of GFIL.
<17> The artificial protein according to any one of <14> to <16>, in which a molecular weight is 1 to 300 kDa.

### Advantageous Effects of Invention

The present invention provides a block copolymer having excellent productivity, a method for producing the same, and an artificial protein having excellent productivity of the block copolymer.

### Brief Description of Drawings

Fig. 1 is a diagram showing experimental results in Example 1.
Fig. 2 is a diagram showing experimental results in Example 2.
Fig. 3 is a diagram showing experimental results in Example 2.
Fig. 4 is a diagram showing experimental results in Example 2.
Fig. 5 is a diagram showing experimental results in Example 3.
Fig. 6 is a diagram showing experimental results in Example 3.
Fig. 7 is a diagram showing experimental results in Example 4.

### Description of Embodiments

Hereinafter, main embodiment of the present invention will be described. However, the present invention is not limited to the explicitly described embodiments.

In the present specification, a numerical range represented by using a symbol "to" means a range including numerical values described before and after "to" as a lower limit value and an upper limit value, respectively.

In the present specification, the term "step" means not only an independent step but also a step that cannot be clearly distinguished from other steps as long as an intended action of the step can be achieved.

In the present specification, unless otherwise specified, the temperature is 23°C, the atmospheric pressure is 101,325 Pa (1 atm), and the humidity (relative humidity) is 50% RH.

In the present specification, a combination of preferable embodiments is a more preferable embodiment.

### (Block Copolymer)

According to the present embodiment, there is provided a block copolymer having a first segment including a protein structure, and one or a plurality of second segments including a molecular group having a function of plasticizing the protein structure and bound to the first segment, in which the protein structure has a main sequence and a hydrophobic tag bound to an N-terminus and a C-terminus of the main sequence, and has at least two reaction sites that react with a compound having the second segment and a reactive group, among the reaction sites, the reaction site closest to the N-terminus side is present within the hydrophobic tag on the N-terminus side, is NH₂ that is the N-terminus of the protein structure, or is present within the main sequence, and among the reaction sites, the reaction site closest to the C-terminus side is present within the hydrophobic tag on the C-terminus side or is present within 9 residues from the C-terminus of the main sequence.

The block copolymer of the present invention is excellent in productivity. The productivity of the block copolymer here can also be regarded as the reactivity between the first segment and a compound having the second segment and a reactive group.

Specifically, it is considered that when the protein structure includes hydrophobic tags at both terminals, and the reaction site closest to the N-terminus side is present within the hydrophobic tag on the N-terminus side, is NH₂ that is the N-terminus of the protein structure, or is present within the main sequence, the reaction site is likely to be exposed on the surface, the reactivity with the compound is enhanced, and as a result, the productivity of the block copolymer as a reaction product is improved. In addition, in a case where the reaction site is one capable of binding protein structures to each other, such as a thiol group, it is also considered that binding between the protein structures to each other at the reaction site is suppressed, whereby the reactivity with the compound is enhanced.

In addition, it is considered that when the reaction site closest to the C-terminus side is present within the hydrophobic tag on the C-terminus side, or is present within 9 residues from the C-terminus of the main sequence, the reaction site is likely to be exposed to the surface, leading to excellent reactivity with the compound.

In addition, when the reaction site closest to the C-terminus side is present within 9 residues from the C-terminus of the main sequence, in order to enhance the reactivity with the compound, the reaction site may be present within 8 residues from the C-terminus of the main sequence, may be present within 7 residues, may be present within 6 residues, may be present within 5 residues, may be present within 4 residues, may be present within 3 residues, may be present within 2 residues, or may be present at the C-terminus of the main sequence. Further, even when the reaction site closest to the N-terminus side is present within the main sequence, in order to enhance the reactivity with the compound, the reaction site may be present within 9 residues from the N-terminus of the main sequence, may be present within 8 residues from the N-terminus, may be present within 7 residues, may be present within 6 residues, may be present within 5 residues, may be present within 4 residues, may be present within 3 residues, may be present within 2 residues, or may be present at the N-terminus of the main sequence.

In addition, in the protein structure included in the block copolymer according to the present embodiment, when the reaction site is a thiol group present at the position described above, there is an advantage that the productivity of the protein structure is particularly improved.

### <First Segment>

### [Protein Structure]

The protein structure includes a main sequence and a hydrophobic tag bound to the N-terminus and the C-terminus of the main sequence. In addition, such a protein structure may also be chemically modified, for example, by acylation of a hydroxyl group of a serine residue, a threonine residue, or a tyrosine residue, an amino group of a lysine residue or the like, or a sulfhydryl group of a cysteine residue. The protein structure chemically modified by such acylation or the like enables introduction of an electrophilic functional group, whereby various functionalities can be imparted.

### [Hydrophobic Tag]

The hydrophobic tag refers to a tag sequence formed from a residue of a hydrophobic amino acid.

However, the C-terminus of the hydrophobic tag may be an amino acid residue including the reaction site (for example, a cysteine residue or a lysine residue), and the N-terminus of the hydrophobic tag may be an amino acid residue including the reaction site (for example, a lysine residue).

Here, the hydrophobic amino acid refers to alanine (A), valine (V), glycine (G), isoleucine (I), leucine (L), phenylalanine (F), proline (P), tryptophan (W), and tyrosine (Y).

That is, in the present disclosure, the hydrophobic tag refers to a tag sequence formed only from a hydrophobic amino acid residue, which is a region in which only a hydrophobic amino acid residue is continuous from a terminal amino acid residue (however, in the case of having a reaction site such as a cysteine residue or a lysine residue at the terminal, the amino acid residue after the terminal), and is a region extending until an amino acid residue that is not hydrophobic (a residue of an amino acid other than the hydrophobic amino acid) is first recognized.

Among them, the hydrophobic tag is preferably a glycine-phenylalanine-isoleucine-leucine (GFIL) sequence or two or more repeats of GFIL, more preferably two or more repeats of GFIL, and more preferably GFILGFIL.

In the above aspect, the N-terminus or the C-terminus of the hydrophobic tag may be an amino acid residue (for example, a cysteine residue or a lysine residue) including the reaction site.

### [Main Sequence]

As the main sequence in the protein structure, a sequence composed of an artificial protein is preferable.

Here, the artificial protein includes a recombinant protein and a synthetic protein. That is, in the present specification, the "artificial protein" means a protein artificially produced. The artificial protein may be a protein having a domain sequence different from an amino acid sequence of a naturally derived protein, or may be a protein having a domain sequence identical to an amino acid sequence of a naturally derived protein. In addition, the "artificial protein" may be a protein obtained by using an amino acid sequence of a naturally derived protein as it is, a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence).

In addition, examples of the artificial protein include proteins that can be used for industrial purposes. The phrase "can be used for industrial purposes" means, for example, being usable for various general-purpose materials used indoors or outdoors. Examples of the artificial protein that can be used for industrial purposes include artificial structural proteins.

### [Artificial Structural Protein]

The structural protein means a protein related to a structure of a living body, a protein constituting a structure created by a living body, or a protein derived from these proteins. The artificial structural protein also refers to a protein that self-aggregates under predetermined conditions to form a structure such as a fiber, a film, a resin, a gel, a micelle, or a nanoparticle. Furthermore, the artificial structural protein can also be said to be a protein in which a motif formed of a characteristic amino acid sequence or a certain number of amino acid residues is repeatedly present to form a skeleton of an organism or a material. In addition, the artificial structural protein is an artificial product of those structural proteins. Examples of such an artificial structural protein include artificial fibroin, artificial keratin, artificial collagen, artificial elastin, and artificial resilin.

When the artificial structural protein is molded, an amino acid having a relatively smaller side chain is more likely to form hydrogen bonds, and a molded body having higher strength is more likely to be obtained. In addition, since the alanine residues and the glycine residues are amino acids having non-polar side chains, the alanine residues and the glycine residues are arranged to face inward in a folding process during the protein production and tend to have an α-helix structure or a β-sheet structure. Therefore, a ratio of an amino acid such as a glycine residue or an alanine residue is desirably high. An alanine residue content only needs to be, for example, 10 to 40%, and may be 12 to 40%, 15 to 40%, 18 to 40%, 20 to 40%, or 22 to 40% from a viewpoint of obtaining a molded body having better strength. A glycine residue content only needs to be, for example, 10 to 55%, and may be 11 to 55%, 13 to 55%, 15 to 55%, 18 to 55%, 20 to 55%, 22 to 55%, or 25 to 55% from a viewpoint of obtaining a molded body having better strength.

In the present specification, the "alanine residue content" means the proportion of the number of alanine residues to the total number of amino acid residues constituting a protein, and is a value represented by the following formula. 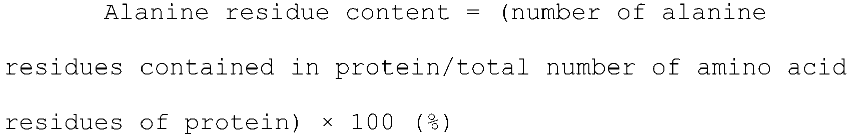 Alanine residue content = (number of alanine residues contained in protein/total number of amino acid residues of protein) × 100 (%)

In addition, a glycine residue content, a serine residue content, a threonine residue content, a proline residue content, and a tyrosine residue content have the same meanings as those obtained by replacing the alanine residue with the glycine residue, the serine residue, the threonine residue, the proline residue, and the tyrosine residue, respectively, in the above formula.

In the artificial structural protein, an amino acid having a relatively large side chain or an amino acid having flexibility is preferably homogeneously contained in the entire sequence to a certain extent. Specifically, the structural protein may include a motif containing a tyrosine residue, a threonine residue, and a proline residue repeatedly in a cycle. When such a structural protein is used, formation of strong intermolecular hydrogen bonds is easily inhibited during processing of a molded body obtained by molding, and processability is easily improved. For example, the sum of the respective contents of a proline residue, a threonine residue, and a tyrosine residue in any consecutive 20 amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. In addition, for example, the sum of the respective contents of a proline residue, a threonine residue, and a tyrosine residue in any consecutive 20 amino acid residues may be 50% or less, 40% or less, 30% or less, or 20% or less.

The artificial structural protein may have an amino acid sequence having a repetitive sequence. That is, the artificial structural protein according to the present embodiment may have a plurality of amino acid sequences (repetitive sequence units) having high sequence identity in the artificial structural protein. The number of amino acid residues of the repetitive sequence unit is preferably 6 to 200. In addition, the sequence identity between the repetitive sequence units may be, for example, 85% or higher, 90% or higher, 95% or higher, 96% or higher, 97% or higher, 98% or higher, or 99% or higher. Further, a hydrophobic degree (average hydropathy index) of the repetitive sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. Note that an upper limit value of the hydrophobic degree of the repetitive sequence unit is not particularly limited, and may be, for example, 1.0 or less or 0.7 or less.

The artificial structural protein may have an amino acid sequence including an (A)ₙ motif. In the present specification, the (A)ₙ motif means an amino acid sequence mainly containing an alanine residue. The number of amino acid residues in the (A)ₙ motif may be 2 to 27 and may be an integer of 2 to 20, 2 to 16, or 2 to 12. In addition, a proportion of the number of alanine residues relative to the total number of amino acid residues in the (A)ₙ motif may be 40% or more, and may be 60% or more, 70% or more, 80% or more, 83% or more, 85% or more, 86% or more, 90% or more, 95% or more, or 100% (which means that the (A)ₙ motif consists only of alanine residues).

The artificial structural protein preferably includes a plurality of (A)ₙ motifs. When a plurality of (A)ₙ motifs are present in the artificial structural protein, the (A)ₙ motifs may have the same amino acid sequence or different amino acid sequences.

In addition, the amino acid sequence including the (A)ₙ motif is preferably different from the amino acid sequence of a naturally derived protein.

Among these, an aspect in which the artificial protein has an amino acid sequence including an (A)ₙ motif, the amino acid sequence is different from the amino acid sequence of a naturally derived protein, the (A)ₙ motif indicates an amino acid sequence composed of 2 to 27 amino acid residues, and the number of alanine residues is 40% or more relative to the total number of amino acid residues in the (A)ₙ motif is also one of preferable aspects in the present embodiment. Preferable aspects of the (A)ₙ motif in the above aspects are as described above.

The artificial structural protein may be an artificial fibroin. Examples of the fibroin include a naturally derived fibroin. Examples of the naturally derived fibroin include a fibroin produced by insects or arachnids. A natural fibroin is a fibrous protein, has a molecular weight of about 370,000, is constituted by two subunits, and has a high content of a glycine residue, an alanine residue, a serine residue, and a tyrosine residue, and these amino acid residues account for nearly 90% of the total number of amino acid residues. The natural fibroin has a crystalline region rich in amino acid residues having relatively small side chains, such as glycine, alanine, and serine, and an amorphous region having amino acid residues having relatively large side chains, such as tyrosine.

More specific examples of the naturally derived fibroin include a fibroin whose sequence information is registered in NCBI GenBank. For example, it can be confirmed by extracting, from sequence information registered in NCBI GenBank, sequences that contain INV as the DIVISION, and further meet the following conditions: a sequence in which "spidroin", "ampullate", "fibroin", "silk and polypeptide", or "silk and protein" is described as a keyword in the DEFINITION; a sequence in which a specific character string for a product is described in the CDS; or a sequence in which a specific character string is described in the TISSUE TYPE under SOURCE.

In the present specification, the "artificial fibroin" means an artificially produced fibroin (artificial fibroin). The artificial fibroin may be a fibroin having an amino acid sequence different from the amino acid sequence of a naturally derived fibroin, or may be a fibroin having an amino acid sequence identical to the amino acid sequence of a naturally derived fibroin. The artificial fibroin can be produced by a known method, for example, a method described in WO 2019/194263 A.

In addition, in a case where the modified fibroin is expressed by the formation of an insoluble matter in the cell, similarly, the host cells are recovered, disrupted and centrifuged to recover the insoluble matter of the modified fibroin as a precipitated fraction. A purified preparation may be obtained by adding citric acid and water to the recovered insoluble matters of the modified fibroin to perform inactivation treatment (for example, 80°C for 2 hours), washing with water by filter press filtration, granulating, and drying.

The artificial fibroin may be a fibrous protein having a structure similar to that of a naturally derived fibroin, or may be fibroin having a sequence similar to a repetitive sequence of the naturally derived fibroin. The "sequence similar to a repetitive sequence of the fibroin" may be an actual sequence of a naturally derived fibroin, or may be a sequence similar thereto.

The "artificial fibroin" may be a fibroin whose amino acid sequence is modified based on a naturally derived fibroin (for example, a fibroin whose amino acid sequence is modified by modifying a cloned gene sequence of a naturally derived fibroin) or a fibroin obtained by artificially designing an amino acid sequence independently of a naturally derived fibroin (for example, a fibroin having a desired amino acid sequence by chemically synthesizing a nucleic acid encoding a designed amino acid sequence) as long as the artificial fibroin has an amino acid sequence specified in the present disclosure. Note that a fibroin obtained by modifying an amino acid sequence of the artificial fibroin is also included in the artificial fibroin as long as an amino acid sequence thereof is different from an amino acid sequence of a naturally derived fibroin. Examples of the artificial fibroin include an artificial silk fibroin (a fibroin obtained by modifying an amino acid sequence of a silk protein produced by silkworms) and an artificial spider silk fibroin (a fibroin obtained by modifying an amino acid sequence of a spider silk protein produced by arachnids). The artificial fibroin preferably contains an artificial spider silk fibroin as a molding material, and more preferably consists of the artificial spider silk fibroin because the artificial spider silk fibroin is relatively easily fibrillated and has a high fiber forming ability.

The artificial fibroin may be a protein having a domain sequence represented by Formula 1: [(A)ₙ motif-REP]ₘ or Formula 2: [(A)ₙ motif-REP]ₘ-(A)ₙ motif. An amino acid sequence (N-terminus sequence and C-terminus sequence) may be further added to any one or both of the N-terminus side and the C-terminus side of the domain sequence of the artificial fibroin. The N-terminus sequence and the C-terminus sequence are typically regions that do not have repeats of amino acid motifs that are characteristic of a fibroin and each consist of about 100 amino acid residues, but are not limited thereto.

In the present specification, the "domain sequence" means an amino acid sequence represented by Formula 1: [(A)ₙ motif-REP]ₘ or Formula 2: [(A)ₙ motif-REP]ₘ-(A)ₙ motif. Here, the (A)ₙ motif represents an amino acid sequence mainly containing alanine residues, and the number of amino acid residues is an integer of 2 to 27. The number of amino acid residues in the (A)ₙ motif may be an integer of 2 to 27, 4 to 27, 4 to 20, 8 to 20, 10 to 20, 4 to 16, 8 to 16, or 10 to 16. In addition, a proportion of the number of alanine residues relative to the total number of amino acid residues in the (A)ₙ motif may be 40% or more, and may be 60% or more, 70% or more, 80% or more, 83% or more, 85% or more, 86% or more, 90% or more, 95% or more, or 100% (which means that the (A)ₙ motif consists only of alanine residues). At least seven (A)ₙ motifs among a plurality of (A)ₙ motifs that are present in the domain sequence may consist only of alanine residues. The REP represents an amino acid sequence consisting of 2 to 200 amino acid residues. The REP may be an amino acid sequence consisting of 10 to 200 amino acid residues. m represents an integer of 2 to 300 and may be an integer of 10 to 300. The plurality of (A)ₙ motifs may be the same amino acid sequence or may be different amino acid sequences. The plurality of REPs may be the same amino acid sequence or may be different amino acid sequences.

Specific examples of the artificial fibroin include an artificial fibroin derived from a major dragline silk protein produced in a major ampullate gland of a spider as described in WO 2019/194263 A (first artificial fibroin), an artificial fibroin having a domain sequence in which the content of glycine residues is reduced (second artificial fibroin), an artificial fibroin having a domain sequence in which the content of an (A)ₙ motif is reduced (third artificial fibroin), an artificial fibroin in which the content of glycine residues and the content of an (A)ₙ motif are reduced (fourth artificial fibroin), an artificial fibroin having a domain sequence including a region locally having a high hydropathy index (fifth artificial fibroin), and an artificial fibroin having a domain sequence in which the content of glutamine residues is reduced (sixth artificial fibroin). Definition of each of the first to sixth artificial fibroins is incorporated herein by reference to the content described in WO 2019/194263 A.

The artificial fibroin may have a tag sequence at one or both of the N-terminus and the C-terminus thereof. This enables isolation, immobilization, detection, visualization, and the like of the artificial fibroin. In addition, the artificial fibroin may have a hinge sequence between the N-terminus of the main sequence and the hydrophobic tag on the N-terminus side.

Examples of the tag sequence include an affinity tag using specific affinity (binding property or affinity) for another molecule. Specific examples of the affinity tag include a histidine tag (His tag). The His tag is a short peptide in which about 4 to 10 histidine residues are lined up and can be used for isolating the artificial fibroin by chelating metal chromatography because the His tag has a property of specifically binding to a metal ion such as nickel. Specific examples of the tag sequence include an amino acid sequence set forth in SEQ ID NO: 8 (an amino acid sequence including a His tag sequence and a hinge sequence).

In addition, a tag sequence such as glutathione-S-transferase (GST) that specifically binds to glutathione or a maltose binding protein (MBP) that specifically binds to maltose can also be used.

Furthermore, an "epitope tag" using an antigen-antibody reaction can also be used. By adding a peptide (epitope) exhibiting antigenicity as a tag sequence, an antibody to the epitope can be bound. Examples of the epitope tag include an HA (a peptide sequence of influenza virus hemagglutinin) tag, a myc tag, and a FLAG tag. By using the epitope tag, the artificial fibroin can be easily purified with high specificity.

Furthermore, a tag sequence that can be cleaved by a specific protease can also be used. By treating a protein adsorbed via the tag sequence with a protease, an artificial fibroin from which the tag sequence is cleaved can be collected.

Specific examples of the artificial fibroin include artificial fibroins set forth in SEQ ID NOs: 1 to 7. The artificial fibroin may be artificial fibroins set forth in SEQ ID NOs: 1 to 7 or artificial fibroin containing an amino acid sequence having a sequence identity of 90% or more with these amino acid sequences. The respective contents of alanine residues, glycine residues, serine residues, threonine residues, tyrosine residues, glutamine residues, and lysine residues of the artificial fibroin shown in SEQ ID NOs: 1 to 7 are shown in Table 1 below. In addition, the artificial fibroin shown in SEQ ID NOs: 1 and 7 corresponds to the fourth artificial fibroin described above, the artificial fibroin shown in SEQ ID NOs: 2, 3, 5, and 6 corresponds to the sixth artificial fibroin described above, and the artificial fibroin shown in SEQ ID NO: 4 corresponds to the first artificial fibroin described above.

**[Table 1]**

| | Alanine residue | Glycine residue | Serine residue | Threonine residue | Tyrosine residue | Glutamine residue | Lysine residue |
|---|---|---|---|---|---|---|---|
| | content (%) | content (%) | content (%) | content (%) | content (%) | content (%) | content (%) |
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7.0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |

The artificial fibroin may be an artificial fibroin having at least two or more of the characteristics of the first artificial fibroin, the second artificial fibroin, the third artificial fibroin, the fourth artificial fibroin, the fifth artificial fibroin, and the sixth artificial fibroin.

A molecular weight of the artificial fibroin according to the present embodiment is not particularly limited, and may be, for example, 2 kDa or more and 700 kDa or less. The molecular weight of the artificial fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 700 kDa or less, 600 kDa or less, 500 kDa or less, 400 kDa or less, less than 360 kDa, 300 kDa or less, or 200 kDa or less.

### [Reaction Site]

The protein structure has at least two reaction sites that react with the compound having the second segment and a reactive group.

In the block copolymer, the reaction site has a structure after reaction with the compound, and serves as a binding point with the second segment.

The reaction site as described above may be, for example, at least one selected from the group consisting of a thiol group, an amino group, a hydroxy group, a guanidino group, a carboxy group, a phenoxy group, an indole group, an amide group, an azide group, and an alkynyl group, preferably at least one selected from the group consisting of a thiol group, an amino group, a hydroxy group, a guanidino group, a carboxy group, a phenoxy group, an indole group, and an amide group, more preferably at least one selected from the group consisting of a thiol group, an amino group, a hydroxy group, a guanidino group, a carboxy group, a phenoxy group, and an indole group, still more preferably at least one selected from the group consisting of a thiol group, an amino group, a hydroxy group, a guanidino group, a carboxy group, and a phenoxy group, still more preferably at least one selected from the group consisting of a thiol group, an amino group, a hydroxy group, a guanidino group, and a carboxy group, still more preferably at least one selected from the group consisting of a thiol group, an amino group, a hydroxy group, and a guanidino group, particularly preferably at least one selected from the group consisting of a thiol group, an amino group, and a hydroxy group, particularly preferably at least one selected from the group consisting of a thiol group and an amino group, and most preferably a thiol group. The amino group may be, for example, an amino group of lysine. The hydroxy group may be, for example, a hydroxy group of serine, a hydroxy group of threonine, or the like. The guanidino group may be, for example, a guanidino group of arginine. The carboxy group may be, for example, a carboxy group of glutamic acid, a carboxy group of aspartic acid, or the like. The phenoxy group may be, for example, a phenoxy group of tyrosine. The indole group may be, for example, an indole group of tryptophan. The amide group may be, for example, an amide group of glutamine, an amide group of asparagine, or the like. The alkynyl group may be an alkynyl group introduced by reacting halogenated acetylene with a thiol group of cysteine.

The number of reaction sites in the protein structure is preferably 2 to 5, more preferably 2 to 4, still more preferably 2 or 3, and particularly preferably 2.

Among the reaction sites, the reaction site closest to the N-terminus side is present within the main sequence, is NH₂ that is the N-terminus of the protein structure, or is present within the hydrophobic tag on the N-terminus side.

Here, the reaction site closest to the N-terminus side among the reaction sites may be present within the hydrophobic tag on the N-terminus side, may be NH₂ that is the N-terminus of the protein structure, or may be present within 9 residues from the terminal (that is, the binding point with the hydrophobic tag on the N-terminus side) of the main sequence; may be present within the hydrophobic tag on the N-terminus side, may be NH₂ that is the N-terminus of the protein structure, or may be present within 8 residues; may be present within the hydrophobic tag on the N-terminus side, may be NH₂ that is the N-terminus of the protein structure, or may be present within 7 residues; may be present within the hydrophobic tag on the N-terminus side, may be NH₂ that is the N-terminus of the protein structure, or may be present within 6 residues; may be present within the hydrophobic tag on the N-terminus side, may be NH₂ that is the N-terminus of the protein structure, or may be present within 5 residues; may be present within the hydrophobic tag on the N-terminus side, may be NH₂ that is the N-terminus, or may be present within 4 residues; may be present within the hydrophobic tag on the N-terminus side, may be NH₂ that is the N-terminus of the protein structure, or may be present within 3 residues; or may be present within the hydrophobic tag on the N-terminus side, may be NH₂ that is the N-terminus of the protein structure, or may be present within 2 residues; or may be present within the hydrophobic tag on the N-terminus side, may be NH₂ that is the N-terminus of the protein structure, or may be present at the N-terminus of the main sequence. In addition, when a hinge sequence is present between the N-terminus of the main sequence and the hydrophobic tag, it is considered that by constituting the hinge sequence with an amino acid having a small side chain, the inhibition of the binding to the compound at the reaction site present at the N-terminus of the main sequence by the side chain of the amino acid constituting the hydrophobic tag can be advantageously suppressed.

Among the reaction sites, the reaction site closest to the C-terminus side is present within the hydrophobic tag on the C-terminus side, or present within 9 residues from the C-terminus of the main sequence.

Here, it is also one preferable aspect of the present invention that the reaction site closest to the C-terminus side among the reaction sites is present at the C-terminus of the hydrophobic tag on the C-terminus side or is present within 9 residues from the C-terminus of the main sequence.

Here, from the viewpoint of the maximum elongation when a fiber, a film, or the like is formed from the block copolymer, the reaction site closest to the C-terminus side among the reaction sites may be present within the hydrophobic tag on the C-terminus side, or may be present within 9 residues from the C-terminus of the main sequence; may be present within the hydrophobic tag on the C-terminus side, or may be present within 8 residues from the C-terminus of the main sequence; may be present within the hydrophobic tag on the C-terminus side, or may be present within 7 residues from the C-terminus of the main sequence; may be present within the hydrophobic tag on the C-terminus side, or may be present within 6 residues from the C-terminus of the main sequence; may be present within the hydrophobic tag on the C-terminus side, or may be present within 5 residues from the C-terminus of the main sequence; may be present within the hydrophobic tag on the C-terminus side, or may be present within 4 residues from the C-terminus of the main sequence; may be present within the hydrophobic tag on the C-terminus side, or may be present within 3 residues from the C-terminus of the main sequence; may be present within the hydrophobic tag on the C-terminus side, or may be present within 2 residues from the C-terminus of the main sequence; or may be present within the hydrophobic tag on the C-terminus side, or may be present at the C-terminus of the main sequence.

In addition, it is also one of preferable aspects of the present invention that the reaction site is present at the C-terminus of the hydrophobic tag.

When the reaction site closest to the N-terminus side in the protein structure is present within the main sequence, and the reaction site closest to the C-terminus side is present within the hydrophobic tag on the C-terminus side, or is present within 9 residues from the C-terminus of the main sequence, for example, all of these reaction sites may be composed of a thiol group.

When such a protein structure is, for example, an artificial protein (recombinant protein) produced by forming an inclusion body in a host cell, an improvement in productivity of the protein structure (recombinant protein) is expected. Although the reason for this is not clear, it is considered that since the protein structure has a hydrophobic tag on the N-terminus side and the C-terminus side of the main sequence, the inclusion body formed in the host cell becomes larger, the recovery rate of the recombinant protein from the host cell is increased, and furthermore, the thiol groups are present at the above positions to be separated from each other.

In addition, when the reaction site closest to the N-terminus side in the protein structure is present within the hydrophobic tag and the reaction site closest to the C-terminus side is present within 9 residues from the C-terminus of the main sequence, for example, all of these reaction sites may be composed of an amino group, and the reaction site closest to the N-terminus side may be present at the N-terminus of the hydrophobic tag.

Specifically, the block copolymer including such a protein structure has an advantage that the bond between the first segment and the compound having the second segment and a reactive group is stably retained for a longer period of time.

When the reaction site on the N-terminus side and the reaction site on the C-terminus side are amino groups, for example, a thiol group (which does not function as a reaction site as referred to in the present embodiment) may be present within 9 residues from the N-terminus of a main sequence (within 9 residues excluding the amino group when an amino group is present at the N-terminus) and may also be present at the C-terminus of the hydrophobic tag on the C-terminus side. In such a protein structure, the reactivity with the compound can be enhanced to enhance the productivity of the block copolymer, and from the above reason, an improvement in productivity of the protein structure itself is also expected.

When the reaction site on the N-terminus side and the reaction site on the C-terminus side are amino groups, it is also preferable that the reaction site closest to the N-terminus side is NH₂ which is the N-terminus of the protein structure, and the reaction site closest to the C-terminus side is a protein structure in which the reaction site is present within the hydrophobic tag on the C-terminus side or within 9 residues from the C-terminus of the main sequence.

Here, NH₂ that is the N-terminus of the protein structure may be, for example, NH₂ of the terminal methionine encoded by the start codon AUG. In addition, when the protein structure is modified, for example, methionine is cleaved by methionyl aminopeptidase (MAP), the amino acid may be another amino acid.

When the reaction site closest to the N-terminus side is NH₂ that is the N-terminus of the protein structure, it is also one of preferable embodiments of the present invention that there is no other reaction site within the hydrophobic tag. According to such an aspect, gelation of the block copolymer may be suppressed.

### [Physical Properties]

A molecular weight of the protein structure may be, for example, 1 kDa or more, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more. The molecular weight of the first segment and the molecular weight of the protein structure included in the first segment may be 400 kDa or less, 360 kDa or less, 300 kDa or less, 250 kDa, 200 kDa, 150 kDa, 100 kDa, 70 kDa, 50 kDa, or 30 kDa or less.

In the present specification, the molecular weight is a value measured by sodium dodecyl sulfate-polyacrylamide gel electrophoresis (SDS-PAGE). The electrophoresis is performed in the following procedure. That is, first, 200 µL of 2 M lithium chloride DMSO (manufactured by FUJIFILM Wako Pure Chemical Corporation) is added to 2 mg of a powder sample, and the mixture is stirred while being heated at 80°C for 60 minutes and further at 95°C for 10 minutes to dissolve the sample. Thereafter, the sample is diluted to 50-fold with a 10 M (mol/L) urea solution, further diluted to 2-fold with a sample buffer (manufactured by FUJIFILM Wako Pure Chemical Corporation), and heated at 95°C for 5 minutes to denature the protein. Subsequently, a gel for SDS-PAGE (manufactured by Bio-Rad) is attached to an electrophoresis apparatus (manufactured by Bio-Rad), the apparatus is filled with an SDS buffer, and the electrophoresis apparatus is connected to a power supply apparatus (manufactured by Bio craft). 10 µL of the denatured sample is added to each well of the gel for SDS-PAGE, and a current of 30 mA/sheet is allowed to flow for 30 minutes. After completion of the electrophoresis, the gel for SDS-PAGE is taken out from the apparatus, immersed in Oriole Fluorescent Gel Stain (manufactured by Bio-Rad), and shaken for one hour. Thereafter, the gel is placed on a UV sample tray (manufactured by Bio-Rad), and a stained image is acquired with a Gel Doc EZ gel imaging apparatus (manufactured by Bio-Rad).

The number of amino acid residues constituting the protein structure may be 50 or more. The number of amino acid residues may be, for example, 100 or more, 150 or more, 200 or more, 250 or more, 300 or more, 350 or more, 400 or more, 450 or more, or 500 or more. The number of amino acid residues may be, for example, 5,000 or less, 4,500 or less, 4,000 or less, 3,500 or less, 3,000 or less, 2,500 or less, 2,000 or less, 1,500 or less, or 1,000 or less. As the number of amino acid residues decreases, solubility in a solvent tends to increase. Therefore, in a case where the number of amino acid residues of the protein structure according to the present embodiment is, for example, 5,000 or less or 2,500 or less, when a polymer compound including a protein structure is obtained by dissolving a compound including a protein structure in a solvent and reacting the compound with a compound including a molecular group having a plasticizing function with respect to the protein structure, it may be desired to improve the generation efficiency of the polymer compound including a target protein structure. In a member such as a film using a polymer compound including a protein structure obtained in this manner, an improvement in flexibility can be expected while securing a certain degree of strength.

The protein structure may be a recombinant protein structure or a hydrophobic protein structure, and may be a hydrophobic recombinant protein structure.

The recombinant protein structure means a protein structure produced using genetic recombination technology. When the protein structure is a recombinant protein structure, the design of the amino acid sequence can be easily changed, which facilitates controlling characteristics, physical properties, and the like of the polymer compound including the protein structure. When the protein structure is a recombinant protein structure, a uniform molecular design is always possible, and thus a protein structure according to a purpose can be stably obtained. This advantageously stabilizes the quality of members such as a polymer compound including a target protein structure and a film.

When the protein structure is a hydrophobic protein structure, its affinity with the first segment of the molecular group having a plasticizing function is improved, and a member such as a film having more excellent flexibility can be produced. In addition, the water resistance of such a member is improved, and for example, in a case where the member is used as a general-purpose material for industrial purposes, the service life can be advantageously extended. In the polymer compound including a protein structure, for example, the hydrophobicity or hydrophilicity of the entire polymer compound including a protein structure can be adjusted in any manner by controlling the hydrophobicity or hydrophilicity of a molecular group included in the second segment and having a plasticizing function with respect to the protein structure. When the protein structure is a hydrophobic protein structure, the entire polymer compound including the protein structure can be shifted to the hydrophobic side as compared with the case where the protein structure is a hydrophilic protein structure, and thus the hydrophobicity or hydrophilicity of the entire polymer compound including the protein structure can be controlled over a wider range.

The hydrophobicity of the hydrophobic protein structure can be determined using a value of the average hydropathy index (hydrophobic degree:hydrophobicity index) described below as an index. The value of the average hydropathy index of the hydrophobic protein structure may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value thereof is not particularly limited, and may be, for example, 1.00 or less or 0.70 or less.

The hydrophobic protein structure preferably has low solubility in a lithium bromide aqueous solution (concentration: 9 M) at 60°C. This solubility can be evaluated using a protein structure obtained by decomposing a compound (protein structure) corresponding to the hydrophobic protein structure or a polymer compound including a protein structure and isolating only the hydrophobic protein structure. In a case where the protein structure is dissolved in a lithium bromide aqueous solution (concentration: 9 M) at 60°C, a maximum concentration may be, for example, less than 30% by mass, less than 25% by mass, less than 20% by mass, less than 15% by mass, less than 10% by mass, less than 5% by mass, or less than 1% by mass. The hydrophobic protein structure may be completely insoluble in a lithium bromide aqueous solution (concentration: 9 M) at 60°C.

The hydrophobic protein structure preferably has a large water contact angle. The contact angle of water can be evaluated by forming a film including a protein structure obtained by decomposing a compound (protein structure) corresponding to the hydrophobic protein structure or a polymer compound including a protein structure on a base material and isolating only the hydrophobic protein structure, and using the film. A protein structure constituting a film such that, when water is dropped onto the film, the contact angle after 5 seconds has elapsed is 55° or greater is preferable as the hydrophobic protein structure. The contact angle may be, for example, 60° or more, 65° or more, or 70° or more.

The hydrophobic protein structure preferably has excellent hot water resistance. The hot water resistance can be evaluated using a protein structure obtained by decomposing a compound (protein structure) corresponding to the hydrophobic protein structure or a polymer compound including a protein structure and isolating only the hydrophobic protein structure. A dispersion consisting of a protein structure and water, which is a dispersion having a content of the protein structure of 5% by mass, is prepared, and a protein that is not decomposed even when the dispersion is heat-treated at 100°C for 5 hours is preferable as the hydrophobic protein structure.

### <Second Segment>

The second segment includes a molecular group having a plasticizing function for the protein structure. The molecular group having the plasticizing function for the protein structure refers to a molecular group in which an intermolecular force between the molecular groups is smaller than that between the protein structures, and when both are mixed, the flexibility of the material can be improved as compared with the protein structure alone. The molecular group having a plasticizing function for the protein structure can also be said to be a molecular group having a melting point or a glass transition temperature lower than that of the protein structure. The molecular group having the plasticizing function for the protein structure may be, for example, polyether, polyester, or polycarbonate. The polyether as the molecular group may be, for example, polyethylene glycol (PEG) or polytetramethylene glycol (PTMG). The plasticizing function can also be said to be a function of improving flexibility or a function of increasing an elongation at break in bending and/or pulling. As the molecular group having the plasticizing function for the protein structure, a molecular group having biodegradability or a molecular group derived from biomass is suitably used. Therefore, it is sufficiently expected that biodegradability and a biovalue are further improved, and furthermore, the producing energy can be further reduced.

The second segment may include a plurality of molecular groups. In addition, the second segment may include a plurality of molecular groups, and the plurality of molecular groups may be linked to each other. The linking between the molecular groups may partially have a branch. Since the linking partially has a branch, the second segment can have a branched structure by the plurality of molecular groups, and a plurality of bonds with the first segment can be formed for each branch. That is, a network structure of the first segment and the second segment can also be formed by introducing a branch into a part of the linking. As described above, the second segment includes the plurality of molecular groups, these molecular groups are linked to each other, and accordingly, the design flexibility of the second segment can be further increased, making it possible to more easily adjust the flexibility of the member such as a film, for example. In a case where the second segment includes only one of the molecular groups, a plurality of second segments may be linked to each other, and at least one of the plurality of second segments may be bound to the first segment. Alternatively, the plurality of second segments may be bound to one first segment. In a case where the plurality of second segments are linked to each other, a branch may be introduced into a part of the linking between the plurality of second segments to form a network of the second segments.

The second segment may contain, for example, a skeleton derived from at least one selected from the group consisting of polyether, polyester, polycarbonate, polyamide, polyol (polyvinyl alcohol or the like), polyolefin, polyacetal, polyketal, poly(meth)acrylate, silicone, polyurethane, polyalkyleneimine, a phenolic resin, a urea resin, a melamine resin, and polysaccharides, and preferably, may contain at least one functional group selected from the group consisting of a polyether group, a polyester group, a polycarbonate group, a polyamide group, a polyol group (polyvinyl alcohol group or the like), and a modified polysaccharide group, and may have at least one functional group selected from the group consisting of an ether group, an ester group, a carbonate group, an amide group, and a modified polysaccharide group. The second segment may have, for example, at least one structural unit selected from the group consisting of structural units having an ether bond, an ester bond, a carbonate ester bond (carbonate bond), an amide group, a siloxane bond, a urethane bond, a urethane bond, and a urea bond, or and it can also be said that the second segment includes at least one structural unit selected from the group consisting of structural units having an alkylene group, a substituted alkylene group, an oxymethylene group, an alkyleneimine group, and a modified polysaccharide group.

Examples of the polyether group include functional groups derived from polyalkylene glycols such as polyethylene glycol, polypropylene glycol, an ethylene oxide/propylene oxide copolymer, and polybutylene glycol (polytetramethylene glycol (PTMG)). When these polyether groups are included in the second segment, the polyether group may be directly linked to a hetero element (O, N, and S) in an ester group, a thioester group, or an amide group of a linker (to be described below) included in the second segment as necessary. Alternatively, the polyether group may be directly linked to a hetero element (O, N, and S) in an ester group, a thioester group, or an amide group of the protein structure included in the first segment. In this case, the entire polyether group is easily separated from the linker and/or the first segment, and as a result, the biodegradation rate of the polyether group can be increased.

Examples of the polyester group include functional groups derived from polyesters such as polylactic acid, poly(3-hydroxybutanoic acid), a polyhydroxybutanoic acid/hydroxyvaleric acid copolymer, a polyhydroxybutanoic acid/4-hydroxybutanoic acid copolymer, a polyhydroxybutanoic acid/hydroxyhexanoic acid copolymer, polytrimethylene terephthalate, a butanediol/long-chain dicarboxylic acid copolymer, polyethylene terephthalate, polybutylene succinate, a polybutylene succinate adipate copolymer, a polybutylene adipate terephthalate copolymer, polycaprolactone, and poly(trimethylene furandicarboxylate) (PTF). Among the polyesters described above, the polyester group is preferably a functional group derived from a polyester classified as a biomass plastic such as polycaprolactone or a biodegradable plastic.

Examples of the polycarbonate group include functional groups derived from polycarbonates having an aliphatic hydrocarbon chain as a main skeleton, such as 1,6-hexanediol polycarbonate, 1,5-pentanediol polycarbonate, and 1,10-decanediol carbonate.

Examples of the polyamide group include functional groups derived from polyamides such as nylon 3, nylon 4, nylon 5, nylon 6, nylon 11, and nylon 610. Among the polyamides described above, the polyamide group is preferably a functional group derived from a polyamide classified as a biomass plastic or a biodegradable plastic.

Examples of the polyol group (polyvinyl alcohol group or the like) include functional groups derived from polyols (polyvinyl alcohols and the like) such as polyvinyl alcohol and an ethylene-vinyl alcohol copolymer. Among the polyols described above, the polyol group is preferably a functional group derived from a polyol classified as a biomass plastic or a biodegradable plastic.

Examples of the modified polysaccharide group include functional groups derived from compounds obtained by chemically modifying cellulose, starch, chitin, chitosan, and the like. Examples of the chemically modified compound include cellulose acetate, ethyl cellulose, starch acetate, hydroxypropylated starch, carboxymethyl chitin, and carboxymethyl chitosan.

The second segment may further include a linker in addition to the molecular group having the plasticizing function for the protein structure. In this case, the molecular group and the protein structure may be bound via the linker. Examples of the linker include a group formed by a reaction between an electrophilic functional group represented by Formulae (1) to (20) described later and the above-described reaction site.

The reaction site in the first segment described above is a segment that reacts with the compound having the second segment and a reactive group.

As the reactive group, electrophilic functional groups represented by Formulae (1) to (20) described later are preferable.

Among them, it is preferable that the reaction site is a thiol group, and the reactive group is a thiol-reactive group.

As the thiol-reactive group, a group having an ethylenically unsaturated bond is preferable, and a group represented by the following Formula (M-1) or (M-2) is more preferable.

A molecular weight of the second segment (the molecular group having the plasticizing function for the protein structure) may be, for example, 200 to 500,000, 300 to 400,000, 350 to 350,000, 400 to 300,000, 500 to 200,000, 600 to 100,000, 700 to 50,000, 800 to 10,000, 900 to 7,500, or 1,000 to 5,000.

When the molecular weight of the second segment is 200 or more, localization of the molecular group having the plasticizing function in the three-dimensional structure of the molecule can be easily suppressed, and a mass ratio of the molecular group having the plasticizing function that is required to be introduced to exhibit a certain level of function can be sufficiently reduced. As a result, there is a possibility that a reduction in reaction time and a lowering of reaction temperature in the production of the polymer compound including a protein structure can be realized.

When the molecular weight of the second segment is 500,000 or less, the molecular weight is within an appropriate range, and a decrease in binding reactivity of the second segment to the first segment is more easily suppressed.

The molecular weight of the second segment is a weight average molecular weight, and is generally determined by a known method using GPC.

The molecular weight of the second segment (molecular group having a plasticizing function for the protein structure) with respect to the molecular weight (protein structure) of the first segment can be appropriately adjusted depending on the application or the like of the polymer compound including the protein structure. The molecular weight of the second segment (when two or more second segments are bound to one first segment, the total molecular weight) is, for example, preferably 1 to 10,000, more preferably 1.5 to 9,000, still more preferably 2 to 8,000, even more preferably 3 to 7,000, yet more preferably 5 to 5,000, further preferably 7 to 3,000, and still further preferably 10 to 2,000, based on the molecular weight of the first segment of 100.

When the molecular weight of the second segment is 1 or more with respect to the molecular weight of the first segment, the flexibility of a member such as a film obtained using a polymer compound including a protein structure can be further enhanced. When the molecular weight of the second segment is 10,000 or less with respect to the molecular weight of the first segment, sufficient plasticity (flexibility) is provided, and the rigidity of the material of the member such as a film can be further improved. When the molecular weight of the second segment with respect to the molecular weight of the first segment is within the above range, it is possible to produce a member, such as a film, having more excellent flexibility.

The molecular weight of the second segment and the molecular weight of the molecular group having the plasticizing function for the protein structure may be, for example, 1.4 or more, 1.5 or more, 1.6 or more, 1.7 or more, 1.8 or more, 1.9 or more, 2.0 or more, 5.0 or more, 10 or more, 20 or more, 30 or more, or 40 or more, when the molecular weight of the first segment or the molecular weight of the protein structure included in the first segment is 100 (preferably, when the molecular weight of the protein structure included in the first segment is 100). An upper limit value thereof is not particularly limited, and may be, for example, 1,000 or less, 800 or less, 600 or less, 400 or less, 200 or less, 100 or less, 80 or less, 70 or less, 60 or less, or 50 or less. When the molecular weight of the first segment is 100, the molecular weight of the second segment is desirably within a range of 1 to 1,000, more desirably within a range of 1 to 800, still more desirably within a range of 1 to 600, even more desirably within a range of 1 to 400, yet more desirably within a range of 1 to 200, and further desirably within a range of 1 to 100, preferably within a range of 1 to 70, more preferably within a range of 1.5 to 60, still more preferably within a range of 1.5 to 50, and particularly preferably within a range of 2.0 to 50. When the ratio between the molecular weight of the first segment (protein structure) and the molecular weight of the second segment (molecular group having a plasticizing function with respect to the protein structure) is a value within the above range, for example, in a member such as a film obtained using a polymer compound including a protein structure, it can be expected to enhance flexibility, stretchability, or the like while sufficiently retaining the characteristics (for example, high mechanical strength) of the first segment due to having the protein structure. A ratio of the molecular weight of the second segment when the molecular weight of the first segment is 100 is calculated based on the weight average molecular weight.

The content ratio of the first segment (protein structure) and the second segment (molecular group having a plasticizing function) in the polymer compound including the protein structure can be appropriately adjusted depending on the application or the like of a member such as a film. Such a content ratio of the first segment when the second segment is 100 in terms of mass ratio may be, for example, 50 or more, 60 or more, 70 or more, 80 or more, 90 or more, 100 or more, 110 or more, 150 or more, 200 or more, 250 or more, 300 or more, 350 or more, 400 or more, 450 or more, 500 or more, 550 or more, or 600 or more. An upper limit value of such a value is not particularly limited, and may be 1,000 or less, 900 or less, 800 or less, or 700 or less. Since the content ratio of the first segment and the second segment in the polymer compound including the protein structure is set to a value within the above range, wasteful use of the second segment is suppressed, and the production cost of the polymer compound including the protein structure can be suppressed. A reduction of the content ratio of the second segment to the first segment in the polymer compound including a protein structure can be advantageously realized by, for example, reducing the molecular weight of the second segment.

In the description of the above embodiment, the linker has been described as a constituent element of the second segment, and may be treated separately from the second segment. The linker can also be treated as a constituent element of the first segment. A molecular weight of the linker moiety is smaller than those of the protein structure and the molecular group, but in a case where the linker is treated separately from the second segment, as for the molecular weight of the above-described second segment, a range obtained by subtracting the molecular weight of the linker moiety is treated as a suitable range of the molecular weight of the second segment. Similarly, in a case where the linker moiety is treated as a constituent element of the first segment, as for the molecular weight of the above-described first segment, a range obtained by adding the molecular weight of the linker moiety is treated as a suitable range of the molecular weight of the first segment.

### <Block Copolymer>

The number of second segments in the block copolymer is one or plural, preferably 2 or more, more preferably 2 to 10, still more preferably 2 to 8, particularly preferably 2 to 6, and most preferably 2 to 4, relative to one first segment.

The block copolymer having the first segment and the second segment may be, for example, a copolymer in which one or a plurality of second segments are bound to one first segment, or may be a copolymer in which a plurality of blocks having the first segment and the second segment bound to the first segment are linked to each other. The block copolymer contained in the polymer compound including a protein structure may be a polymer (for example, a graft polymer or the like) having the first segment including the protein structure as a main chain and the second segment as a side chain. The block copolymer may contain, for example, a plurality of first segments, and the first segment and the second segment may be alternately bound. By alternately bonding the first segment and the second segment, for example, the elongation and toughness of members such as fibers and films can be further improved.

The block copolymer may have a plurality of the first segments, and a part of the second segments may be bound to two or more of the first segments to form a network structure. By having the network structure, the elongation and toughness of members such as fibers and films can be further improved.

The first segment and the second segment may be directly bound to each other, or may be bound to each other through a structure capable of binding to the first segment and the second segment. In any of these binding aspects, the bond between the first segment and the second segment may be a coordinate bond, may be a bond by an ionic interaction, or may be a covalent bond. Among them, a covalent bond is desirable.

### [Method for Producing Block Copolymer]

According to the present embodiment, there is provided a method for producing a block copolymer, the method including binding a compound having a first segment including a protein structure, and a compound having a second segment including a molecular group having a function of plasticizing the protein structure, in which the protein structure has a main sequence and a hydrophobic tag bound to an N-terminus and a C-terminus of the main sequence, and has at least two reaction sites that react with a compound having the second segment and a reactive group, in which among the reaction sites, the reaction site closest to the N-terminus side is present within the hydrophobic tag on the N-terminus side, is NH₂ that is the N-terminus of the protein structure, or is present within the main sequence, and in which among the reaction sites, the reaction site closest to the C-terminus side is present within the hydrophobic tag on the C-terminus side or is present within 9 residues from the C-terminus of the main sequence.

Preferable aspects of the first segment and the second segment in the method for producing a block copolymer according to the present embodiment are the same as the preferable aspects of the first segment and the second segment in the block copolymer according to the present embodiment described above.

In addition, preferable aspects of the block copolymer produced by the method for producing a block copolymer according to the present embodiment are the same as the preferable aspects of the block copolymer according to the present embodiment described above.

In the method for producing a block copolymer according to the present embodiment, as described above, the reactivity of the compound having the first segment including the protein structure is high. Therefore, the bonding between the compound having the first segment including the protein structure and the compound having the second segment including a molecular group having a function of plasticizing the protein structure rapidly proceeds (reaction rate is high), thereby providing a method having excellent productivity of the block copolymer.

In addition, according to the present embodiment, preferably, the method for producing a block copolymer includes adding the compound having the first segment and the compound having the second segment, to a solvent, and the compound having the second segment is added in a non-solution state.

The compound having the second segment is preferably a compound having the second segment and a reactive group. Preferable aspects of such a compound are as described above.

The non-solution state refers to a state of not being dissolved in a solvent, and is preferably a solid state or a liquid state. In addition, an aspect in which the compound having the second segment is present alone in a non-solution state, rather than as a mixture with other additives or the like, is also one of preferable aspects of the present invention.

According to the above aspect, a block copolymer can be produced by adding the compound having the first segment and the compound having the second segment to one reaction vessel.

The compound having the first segment according to the present embodiment has high reactivity, and the amount of reaction between the compounds having the first segment themselves is small. Therefore, even when the compound having the second segment is added in a non-solution state as described above, the reaction can rapidly proceed.

In the above aspect, adding a reducing agent, a compound having the first segment, and a compound having the second segment, to the solvent in this order is one of the preferable aspects.

### - Reducing Agent -

The reducing agent may contain, for example, at least one selected from the group consisting of a thiol, a dithiol, sodium carbonate, sodium sulfite, sodium hyposulfite, sodium sulfate, and sodium dithionite, and the reducing agent may contain at least one selected from the group consisting of a dithiol, sodium carbonate and sodium sulfite, and may be a dithiol or sodium sulfite. The reducing agent is preferably a compound having a mercapto group.

The compound having a mercapto group is desirably a compound represented by the following general Formula (Y). In the case of such a compound, a chemically stable six-membered ring structure is formed in the oxidized form after the reduction reaction, the reaction between the reducing agent and the reactive group in the compound having the second segment and a reactive group is suppressed from occurring in the reaction system, and for example, the formation of a bond such as a disulfide bond between proteins is suppressed by forming a cyclic structure between the reducing agent and the reaction site of the protein, the reaction between the mercapto group of the protein and the reactive group is made significant, and accordingly, a target polymer compound in accordance with the reaction design can be more efficiently produced. Examples of the dithiol include dithiothreitol and 1,4-butanedithiol. The compound (dithiol) represented by general Formula (Y) is specifically dithiothreitol or 1,4-butanedithiol.

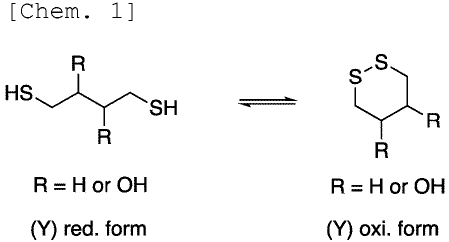

Examples of other compounds having a mercapto group include 3-mercaptopropionic acid, 3-mercapto-1,2-propanediol, and pentaerythritol tetra(3-mercaptopropionate). As the reducing agent, in addition to the above compounds, for example, (tris(2-carboxyethyl)phosphine (TCEP) or the like may be used.

The lower limit value of the blending amount of the reducing agent may be, for example, 0.5 equivalents or more, 0.8 equivalents or more, or 1.0 equivalents or more, relative to the reaction site of the compound having the first segment. By setting the lower limit value of the blending amount of the reducing agent within the above range, the formation of bonds such as a disulfide bond by a reaction between the reaction sites can be more sufficiently inhibited. This can further improve production efficiency of a target synthetic polymer compound. An upper limit value of the blending amount of the reducing agent may be, for example, 2.4 equivalents or less, 2.0 equivalents or less, 1.6 equivalents or less, 1.2 equivalents or less, or 1.1 equivalents or less, relative to the reaction sites. By setting the upper limit value of the blending amount of the reducing agent within the above range, the reaction can be allowed to proceed while further suppressing the reaction between the reactive group and the reducing agent and the generation of by-products.

### - Other Additives -

In the method for producing a block copolymer of the present invention, when the compound having the first segment including the protein structure and the compound having the second segment including the molecular group having the function of plasticizing the protein structure are bound to each other, other additives may be present.

Examples of other additives include a base and a polymerization inhibitor.

### <<Base>>

By containing a base, the reaction site can be activated to promote the reaction with the reactive group.

Examples of the base include a primary amine, a secondary amine, a tertiary amine, a nitrogen-containing cyclic compound, a nitrogen-containing aromatic compound, and an inorganic base. Examples of the primary amine include ethanolamine and hexamethylenediamine. Examples of the secondary amine include diethylamine, methylethylamine, and N-methylbutylamine. Examples of the tertiary amine include triethylamine, diethylmethylamine, and N,N-diisopropylethylamine (DIPEA). Examples of the nitrogen-containing cyclic compound include quinuclidine, 1,4-diazabicyclo[2.2.2]octane (DABCO), 1,8-diazabicyclo[5.4.0]undec-7-ene (DBU), and 1,5-diazabicyclo[4.3.0]non-5-ene (DBN). Examples of the nitrogen-containing aromatic compound include pyridine and imidazole. Examples of the inorganic base include sodium hydroxide, potassium hydroxide, sodium hydrogen carbonate, potassium hydrogen carbonate, sodium carbonate, potassium carbonate, cesium carbonate, tripotassium phosphate, sodium acetate, and potassium acetate.

A lower limit value of the blending amount of the base may be, for example, 0.01 parts by mass or more, 0.05 parts by mass or more, 0.1 parts by mass or more, or 0.5 parts by mass or more, based on the mass of the compound having the first segment. By setting the lower limit value of the blending amount of the base within the above range, the reaction can be further promoted. An upper limit value of the blending amount of the base may be, for example, 50 parts by mass or less, 20 parts by mass or less, 10 parts by mass or less, or 5 parts by mass or less based on the mass of the compound having the first segment. By setting the upper limit value of the blending amount of the base within the above range, decomposition of a molecule due to an influence of the remaining base can be further suppressed, and a target polymer compound can be synthesized. The blending amount of the base may be adjusted within the above-described range, and may be, for example, 0.05 to 50 parts by mass or 0.1 to 10 parts by mass, based on the mass of the compound having the first segment.

### <<Polymerization Inhibitor>>

The polymerization inhibitor is a compound that inhibits the self-polymerization of the compound having the second segment and a reactive group. Examples of the polymerization inhibitor include hydroquinone, methoquinone, 4-tert-butylpyrocatechol, tertbutylhydroquinone, 1,4-benzoquinone, dibutylhydroxytoluene, mequinol, phenothiazine, and 1,1-diphenyl-2-picrylhydrazyl.

The content of the polymerization inhibitor may be, for example, 0.01 parts by mass or more, 0.05 parts by mass or more, 0.1 parts by mass or more, or 0.2 parts by mass or more, relative to 100 parts by mass of the compound having the second segment and a reactive group.

The content of the polymerization inhibitor may be, for example, 25 parts by mass or less, 10 parts by mass or less, 5 parts by mass or less, or 1 part by mass or less, relative to 100 parts by mass of the compound having the second segment and a reactive group. By setting the content of polymerization inhibitor within the above range, inhibition of the reaction by polymerization inhibitor in the system can be further suppressed.

In addition, since the amount of the compound having the first segment reacted between the compounds having the first segment is small, it is also possible to employ an aspect in which the amount of the reducing agent is reduced.

That is, the method for producing a block copolymer according to the present embodiment can be a simple step using only one reaction vessel.

Here, in the conventional method for producing a block copolymer, a method of adding a solution obtained by dissolving the compound having the second segment in a solvent to a solution obtained by dissolving the compound having the first segment in a solvent has been adopted. In such a method, two vessels are required, a vessel for dissolving the compound having the first segment in a solvent and a vessel for dissolving the compound having the second segment in a solvent.

Considering an actual mass production process, the fact that the number of vessels for reaction or preparation can be reduced can be an industrially very important improvement in terms of facilitating the design of the process and apparatus.

### - First Aspect of Method for Producing Block Copolymer -

The block copolymer is preferably obtained by a production method including a step of subjecting a mixture containing a compound having the first segment including the protein structure (hereinafter also referred to as "compound A") and a compound having the second segment including the molecular group having a function of plasticizing the protein structure (hereinafter also referred to as "compound B"), to a mechanochemical treatment.

The mixture may be prepared by mixing a solution containing the compound A and a solvent with a solution containing the compound B and a solvent, or may be prepared by sequentially adding the compound A and the compound B to the solvent in a non-solution state as described above.

The mixture may further contain the above-described reducing agent, base, polymerization inhibitor, and the like.

The above-described first segment is formed by the compound A, and the above-described second segment is formed by the compound B.

That is, the block copolymer is preferably obtained by subjecting a mixture containing the compound including the protein structure and the compound including the molecular group capable of plasticizing the protein structure to a mechanochemical treatment.

Preferable aspects of the protein structure in the compound A are as described above.

The compound B is preferably the compound having the second segment and a reactive group. Preferable aspects of such a compound are as described above.

The reactive group in the compound having the second segment and a reactive group is, for example, a group represented by Formulae (1) to (20). A preferable reactive group is a group represented by Formula (1), (2), (6) to (8), (16), or (19), and a more preferable reactive group is a group represented by Formula (1), (2), or (6). Preferable combinations of the reaction site (a reaction site included in the protein structure in the compound A) and the reactive group are a combination of a hydroxyl group and a hydroxy-reactive group represented by any one of Formulae (2) to (9), (13) to (17), and (19), a combination of an amino group and an amine-reactive group represented by any one of Formulae (2) to (9), (13) to (17), and (19), and a combination of a thiol group and a thiol-reactive group represented by any one of formulas (1) to (6), (8), (10) to (15), and (18).

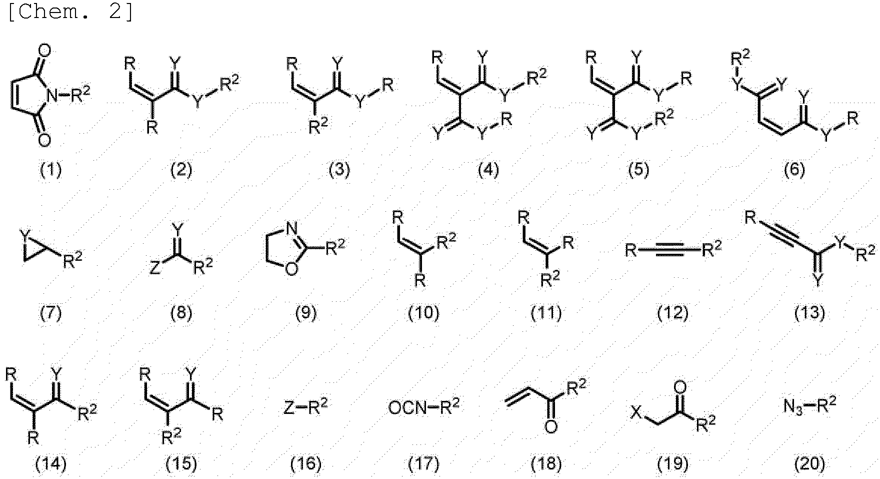

In Formulae (1) to (20), R² represents the partial structure capable of plasticizing the protein. In Formulae (2) to (8) and (13) to (15), Y's each independently represent an oxygen atom, a sulfur atom, or NR¹, and R¹ represents a hydrogen atom, a hydrocarbon group, an aromatic group, a carbonyl group, or a sulfonyl group. In Formulae (2) to (6) and (10) to (15), R's each independently represent a hydrogen atom, a hydrocarbon group, or an aromatic group. In Formulae (8) and (16), Z represents a halogen atom, a sulfonic acid ester group, or a fluorine-containing carboxylic acid ester group. In Formula (19), X represents a halogen atom.

The amount of the compound B used can be 1.5 to 7 equivalents per nucleophilic functional group contained in the compound A. The amount of the compound B used may preferably be 1.5 to 6.5 equivalents, 1.5 to 6 equivalents, 1.5 to 5.5 equivalents, 1.5 to 5 equivalents, 1.5 to 4 equivalents, 2 to 7 equivalents, 2 to 6.5 equivalents, 2 to 6 equivalents, 2 to 5.5 equivalents, 2 to 5 equivalents, 2 to 4.5 equivalents, 2.5 to 7 equivalents, 2.5 to 6.5 equivalents, 2.5 to 6 equivalents, 2.5 to 5.5 equivalents, 2.5 to 5 equivalents, or 2.5 to 4.5 equivalents per nucleophilic functional group. The "equivalent per nucleophilic functional group" means a molar equivalent of the compound B relative to each nucleophilic functional group contained in the compound A. By setting the amount of the compound B used to a value within the above range, the reactivity or reaction efficiency of the compound A and the compound B in the mechanochemical treatment is enhanced, and the target block copolymer is more efficiently obtained.

The mechanochemical treatment is a treatment of performing a chemical reaction caused by directly absorbing mechanical energy. The mechanical energy may be exerted by, for example, an impact force or a shear force. Specifically, such a mechanochemical treatment is performed using a rolling ball mill, a medium stirring mill, a planetary mill, a jet mill, a mixer mill, an extruder (twin-screw extruder), or the like. Specifically, the compound A and the compound B are put in a grinding jar, and a medium ball or the like is put therein depending on the type of mill to be used. Thereafter, the grinding jar is set in a mixer mill apparatus and vibrated at a predetermined frequency and for a predetermined reaction time. At this time, a solvent, a base, a reaction accelerator, and the like may be further added to the grinding jar. Alternatively, the compound A and the compound B are put in a mixer mill and a milling procedure is initiated to knead the reactants continuously. At this time, a solvent, a base, a reaction accelerator, and the like may be further added to the mixer mill. In addition, the compound A and the compound B are charged into an extruder and kneaded. At this time, a solvent, a base, a reaction accelerator, and the like may be further added to the extruder. Note that, when the mechanochemical treatment is performed using the extruder, a target block copolymer can be continuously produced.

The frequency can be appropriately adjusted by a person skilled in the art depending on reaction, and may be, for example, 10 to 50 Hz, 10 to 45 Hz, 10 to 40 Hz, 10 to 35 Hz, 15 to 50 Hz, 15 to 45 Hz, 15 to 40 Hz, 15 to 35 Hz, 20 to 50 Hz, 20 to 45 Hz, 20 to 40 Hz, or 20 to 35 Hz.

A reaction time can be up to a time point at which the compound A serving as a raw material disappears or detection of a block copolymer is observed at a certain level or more in an infrared (IR) absorption spectrum, gel permeation chromatography (GPC), or the like. The reaction time may be any time, and may be, for example, 30 to 240 minutes, 30 to 210 minutes, 30 to 180 minutes, 30 to 150 minutes, 30 to 120 minutes, 30 to 110 minutes, 30 to 100 minutes, 30 to 95 minutes, 60 to 240 minutes, 60 to 210 minutes, 60 to 180 minutes, 60 to 150 minutes, 60 to 120 minutes, 60 to 110 minutes, 60 to 100 minutes, 60 to 95 minutes, 70 to 240 minutes, 70 to 210 minutes, 70 to 180 minutes, 70 to 150 minutes, minutes, 70 to 120 minutes, 70 to 110 minutes, 70 to 100 minutes, 70 to 95 minutes, 80 to 240 minutes, 80 to 210 minutes, 80 to 180 minutes, 80 to 150 minutes, 80 to 120 minutes, 80 to 110 minutes, 80 to 100 minutes, or 80 to 95 minutes.

The solvent may be a solvent capable of swelling the compound A or a solvent capable of dissolving at least one of the compound A and the compound B, but the solvent must be a compound that is liquid at normal temperature and normal pressure and does not chemically react with the compound A. A mechanochemical treatment to which such a solvent is added is known as liquid assisted grinding (LAG), and is a method for allowing a desired reaction to proceed more efficiently by adding a small amount of solvent into a grinding jar. Examples of the solvent include an alcohol-based solvent such as methanol or ethanol, and an aprotic polar solvent such as dimethyl sulfoxide (DMSO), N,N-dimethylformamide (DMF), N,N-dimethylacetamide (DMAc), N-methylpiperidone (NMP), or dihydrolevoglucosenone. The amount of the solvent used may be, for example, 0.01 g to 1 g, and may be 0.01 g to 0.8 g, 0.01 g to 0.6 g, 0.01 g to 0.4 g, or 0.01 g to 0.2 g relative to 1 g of mass of the compound A. In other words, the amount of the solvent used may be, for example, 1 to 100% by mass, and may be 1 to 80% by mass, 1 to 60% by mass, 1 to 40% by mass, or 1 to 20% by mass, relative to the compound A. It is considered that an extremely small amount of the solvent forms a microscopic reaction field by swelling the compound A, locally bringing the compound A into a dissolved state, or the like. Note that addition of the solvent is not necessarily required, and for example, when the compound B is a liquid at normal temperature and normal pressure and has the above function as the solvent, the addition of the solvent may be unnecessary.

After completion of the reaction, the mixture may be removed from the grinding jar, and washed with a solvent. By washing, an unreacted substance, excess by-products generated by the reaction, and the like can be removed. Examples of a solvent used for washing include water, methanol, ethanol, acetonitrile, acetone, tetrahydrofuran, ethyl acetate, and hexane. After washing, the solvent used for washing may be distilled off by drying a product. Drying may be performed under reduced pressure.

An average particle diameter of the block copolymer according to the present embodiment is preferably 1 to 80 µm, 1 to 50 µm, 2 to 50 µm, 2 to 40 µm, 4 to 25 µm, 5 to 25 µm, 8 to 25 µm, or 8 to 16 µm. When the average particle diameter is in the above range, handleability is excellent. The average particle diameter is determined, for example, by the following method. "After particles or powder of the block copolymer is uniformly dispersed on a glass plate by suction of a vacuum chamber, measurement is performed five times each using a dry and wet image analysis particle size distribution analyzer (trade name: DW-200nano, manufactured by JASCO International Co., Ltd.), and a projection image is captured with a 10 million pixel camera, followed by analyzing the obtained projection image using image analysis software.

The block copolymer according to the present embodiment is obtained as a powder having a smaller and more uniform average particle diameter by mechanochemically treating a mixture containing the compound A and the compound B and then freeze-drying the mixture. An average particle diameter of a block copolymer powder obtained by freeze drying is, for example, about 1 to 30 µm, 1 to 20 µm, 1 to 10 µm, or 2 to 8 µm. Furthermore, such a powdery block copolymer has excellent handleability, and the use amount thereof can be finely adjusted. In addition, the freeze-dried block copolymer not only has a small average particle diameter but also is expected to disentangle the tertiary structure of the compound A, and therefore, can be expected to improve dispersibility in an aqueous medium (aqueous liquid) such as water, a basic aqueous solution, an acidic aqueous solution, or a neutral aqueous solution containing an inorganic salt, and solubility in a solvent. In particular, when an aqueous dispersion obtained by dispersing a block copolymer powder obtained by a mechanochemical treatment or a block copolymer powder obtained by further freeze-drying the block copolymer powder in an aqueous medium is applied onto a surface of a base material, the aqueous dispersion can be more uniformly applied without unevenness. As a result, a more uniform coating film having as little unevenness as possible can be formed. That is, the aqueous dispersion of the block copolymer powder can also be useful as a coating liquid for forming a coating film. In addition, by applying an aqueous dispersion of the block copolymer powder onto a surface of a base material, bonding another base material thereto, and then curing the block copolymer, the two base materials can also adhere to each other. In other words, the aqueous dispersion of the block copolymer according to the present embodiment can also be useful as a water-dispersible adhesive. Note that, when the aqueous dispersion of the block copolymer is used as a coating liquid, for example, a predetermined colorant or an additive generally used for a coating liquid or the like may be added to, blended with, or mixed with the aqueous dispersion of the block copolymer. In addition, when the aqueous dispersion of the block copolymer is used as a water-dispersible adhesive, an additive generally used for an adhesive or the like may be added to, blended with, or mixed with the aqueous dispersion of the block copolymer. The coating liquid or the water-dispersible adhesive containing the aqueous dispersion of the block copolymer according to the present embodiment uses an aqueous medium such as water as a solvent, and is therefore suitable for application to a material having low organic solvent resistance and adhesion of a material having low organic solvent resistance, and can reduce odor peculiar to an organic solvent.

In a block copolymer produced by a mechanochemical method, an organic solvent is not used in a production process thereof, or only an extremely small amount (an amount sufficient to swell a molecule capable of plasticizing the compound A or the compound B) of an organic solvent is used, and therefore the organic solvent is less likely to remain, and the block copolymer is easily purified as a powder.

### - Second Aspect of Method for Producing Block Copolymer -

In addition, it is also preferable that the block copolymer in the polymer compound including a protein structure is produced by a method including heating and reacting a mixture containing a compound, which is the compound having the above-described first segment and in which the above-described reaction site is a mercapto group (hereinafter also referred to as "compound C"), a compound having at least one structure selected from the group consisting of polyethers, polyesters, and polycarbonates having two structures represented by the following Formula (M-1) or (M-2) (hereinafter also referred to as "compound D"), and an organic solvent.

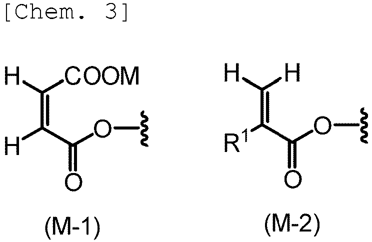

In Formula (M-1), M represents any one of H, Na, K, NHEt₃, and NHEtiPr₂. In the present specification, Et represents an ethyl group, and iPr represents an isopropyl group.

In Formula (M-2), R¹ represents a hydrogen atom or a methyl group.

The mixture may be prepared by mixing a solution containing the compound C and the solvent with a solution containing the compound D and the solvent, or may be prepared by sequentially adding the compound C and the compound D to the solvent in a non-solution state as described above.

The mixture may further contain the above-described reducing agent, base, polymerization inhibitor, and the like.

The above-described first segment is formed from the compound C, and the above-described second segment is formed from the compound D.

### <<Compound C>>

The compound C is a compound having the above-described first segment. Here, the mercapto group in the compound C is preferably a mercapto group included in a cysteine residue in the protein structure.

In addition, the preferable aspect of the first segment in the compound C is as described above.

In the production method, the blending amount of the compound C may be, for example, more than 1 part by mass, 2 parts by mass or more, 5 parts by mass or more, more than 5 parts by mass, 6 parts by mass or more, 7 parts by mass or more, or 8 parts by mass or more, relative to 100 parts by mass of organic solvent. According to the production method according to the present disclosure, the blending amount of the compound C may be, for example, 50 parts by mass or less, 30 parts by mass or less, 20 parts by mass or less, or 15 parts by mass or less, relative to 100 parts by mass of dimethyl sulfoxide. When an upper limit value of the blending amount of the compound C is within the above range, the reaction can be allowed to proceed while more sufficiently suppressing generation and gelation of a by-product.

### <<Compound D>>

Preferable aspects of the polyether, polyester, and polycarbonate in the compound D are the same as the preferable aspects thereof in the above-described second segment.

In the production method, a ratio of the number of moles of the structure represented by Formula (M-1) or (M-2) and included in the compound D to the number of moles of mercapto groups of the compound C may be, for example, 0.1 times or more, 0.2 times or more, 0.5 times or more, or 0.7 times or more. By setting the ratio within the above range, the reaction can be allowed to proceed while further suppressing a decrease in reactivity.

In addition, in the production method, the ratio of the number of moles may be, for example, 5.0 times or less, 3.0 times or less, 1.5 times or less, or 0.8 times or less. By setting the ratio within the above range, the reaction can be allowed to proceed while further suppressing generation and gelation of a by-product. By adjusting the molar ratio, the number of polyether structures and the like in the obtained polymer compound can be adjusted.

### <<Organic Solvent>>

The organic solvent is not particularly limited as long as it can dissolve the compound C and the compound D, but dimethyl sulfoxide is preferable.

Here, when a compound having a structure represented by the above-described Formula (M-1) is used as the compound D, a reducing agent is preferably contained during the heating reaction, and a reducing agent and a base are preferably contained during the heating reaction.

In addition, when a compound having a structure represented by the above-described Formula (M-2) is used as the compound D, a polymerization inhibitor and a reducing agent are preferably contained during the heating reaction, and a polymerization inhibitor, a reducing agent, and a base are preferably contained during the heating reaction.

Preferable aspects of the reducing agent, the base, and the polymerization inhibitor are as described above.

### <<Reaction Conditions>>

In the above production method, the reaction temperature in the heating reaction may be, for example, 50°C or higher, 53°C or higher, 55°C or higher, 57°C or higher, or 60°C or higher. When the lower limit value of the reaction temperature is within the above range, the reaction between the compound C and the compound D can be further promoted. In the above production method, the upper limit value of the reaction temperature of the reaction between the compound C and the compound D may be, for example, 90°C or less, 85°C or less, 80°C or less, 75°C or less, or 70°C or less. When the upper limit value of the reaction temperature is within the above range, formation of bonds between mercapto groups of the protein as a side reaction can be further suppressed, and a target polymer compound can be efficiently synthesized. The reaction temperature of the reaction between the compound C and the compound D may be adjusted within the above-described range, and may be, for example, 50 to 90°C, 53 to 80°C, 55 to 75°C, or 55 to 70°C.

The timing of heating is not limited to after mixing the compound C and the compound D, and further, components such as a base, a reducing agent, and a polymerization inhibitor added as necessary. For example, these components may be added using an organic solvent (for example, dimethyl sulfoxide) heated in advance. Heating may be started after adding necessary components among components such as a base, a reducing agent, and a polymerization inhibitor to dimethyl sulfoxide, and after reaching a predetermined temperature, the compound C and the compound D may be added and reacted. Heating may be started after adding necessary components among components such as a base, a reducing agent, and a polymerization inhibitor to one of the compound C and the compound D, and after reaching a predetermined temperature, the remaining one of the compound C and the compound D may be added and reacted.

According to such a method, the block copolymer is obtained in the state of a solution in which the block copolymer is dissolved in an organic solvent.

From this solution, a block copolymer is recovered by a known method such as drying, reprecipitation, or dialysis, and pulverized as necessary, whereby a powder of a polymer compound including a protein structure can be obtained.

In addition, the block copolymer can also be obtained by, for example, the method described in WO 2023/013638 A.

### <Application>

The block copolymer in the present embodiment can be used for synthetic leather, electronic materials, and the like, and is preferably used as synthetic leather.

As the synthetic leather, for example, the synthetic leather can be used for the same application as the conventional synthetic leather (for example, synthetic leather made of synthetic resin). The synthetic leather according to the present embodiment can be used for, for example, in applications such as clothing, shoes, accessories such as bags, various covers, furniture, and automobile interior materials.

The electronic material can be used, for example, in applications including flexible flat cables, flexible printed wiring boards, insulating films, cover films, reinforcing plates, glass, support members such as glass and silicon wafers.

### (Artificial Protein)

According to the present invention, there is provided an artificial protein including a main sequence and a hydrophobic tag bound to an N-terminus and a C-terminus of the main sequence, and the artificial protein including hydrophobic tags at the N-terminus and the C-terminus, in which the artificial protein contains at least two cysteine residues, among the cysteine residues, a cysteine residue closest to the N-terminus side is present within the main sequence, and among the cysteine residues, a cysteine residue closest to the C-terminus side is present within the hydrophobic tag on the C-terminus side or present within 9 residues from the C-terminus of the main sequence.

The preferable aspect of the artificial protein of the present invention is the same as the preferable aspect of the protein structure in the block copolymer of the present invention described above, except that the reaction site is limited to a cysteine residue (thiol group).

By using the artificial protein of the present invention, the block copolymer of the present invention can be produced with high productivity.

In addition, according to the present invention, there is provided an artificial protein having a main sequence and a hydrophobic tag bound to an N-terminus and a C-terminus of the main sequence, and the artificial protein including hydrophobic tags at the N-terminus and the C-terminus, in which the artificial protein contains at least one lysine residue, among the lysine residues, a lysine residue closest to the N-terminus side is present within the hydrophobic tag on the N-terminus side, or no lysine residue is present within the hydrophobic tag on the N-terminus side; and among the lysine residues, a lysine residue closest to the C-terminus side is present within the hydrophobic tag on the C-terminus side or present within 9 residues from the C-terminus of the main sequence.

When there is no lysine residue within the hydrophobic tag on the N-terminus side, NH₂ present at the terminal of the artificial protein can serve as a reaction site.

The preferable aspect of the artificial protein of the present invention is the same as the preferable aspect of the protein structure in the block copolymer of the present invention described above, except that the reaction site is limited to a lysine residue (amino group).

By using the artificial protein of the present invention, the block copolymer of the present invention can be produced with high productivity.

In addition, even when the reaction site is limited to a lysine residue, a cysteine residue that does not function as the reaction site may be included.

### Examples

Hereinafter, the present invention will be described more specifically with reference to Examples. Materials, amounts used, proportions, processing contents, processing procedures, and the like shown in the following examples can be appropriately changed without departing from the gist of the present invention. Therefore, the scope of the present invention is not limited to the following specific examples.

Hereinafter, M represents "mol/L", mM represents "mmol/L", and µM represents "µmol/L".

### (Example 1: Examination of Protein Production Amount and Reactivity)

### <Production of Protein (Protein CTR) Containing GFIL Sequence at One Terminal on C-Terminus Side>

### [Preparation of Polypeptide]

### (Preparation of Expression Vector)

An artificial fibroin having the amino acid sequence set forth in SEQ ID NO: 9 (PRT2882) was designed.

Note that an average hydropathy index value of the artificial fibroin having the amino acid sequence set forth in SEQ ID NO: 9 (PRT2882) is 0.47.

Nucleic acids encoding the artificial fibroin having the amino acid sequence set forth in SEQ ID NO: 9 were each synthesized. In each of the nucleic acids, an NdeI site was added to a 5' terminal and an EcoRI site was added downstream of a stop codon. The nucleic acid was cloned into a cloning vector (pUC118). Thereafter, the nucleic acid was subjected to restriction enzyme treatment with NdeI and EcoRI to excise a fragment, and then, recombined into a polypeptide expression vector pET-22b(+), thereby obtaining an expression vector.

### (Preparation of Polypeptide)

The obtained expression vector was used to transform *Escherichia coli* BLR(DE3). The transformed *Escherichia coli* was cultured in 2 mL of an LB culture medium containing ampicillin for 15 hours. The culture solution was added to a 100 mL seed culture medium containing ampicillin (Table 2) such that the absorbance OD₆₀₀ at a wavelength of 600 nm was 0.005. While the temperature of the culture medium was maintained at 30°C, flask culture was conducted until the OD₆₀₀ reached 5 (about 15 hours) to obtain a seed culture medium.

**[Table 2]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter containing 500 mL of a production medium (Table 3) such that the OD₆₀₀ was 0.05. The culture was carried out while keeping the culture solution temperature at 37°C and controlling the pH constant at 6.9. Furthermore, the dissolved oxygen concentration in the culture solution was also maintained at 20% of the dissolved oxygen saturation concentration.

**[Table 3]**

| Production medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| ADEKA NOL (ADEKA, LG-295S) | 0.1 (mL/L) |

Immediately after glucose in the production medium was completely consumed, a feed solution (455 g/1 L of glucose, 120 g/1 L of Yeast Extract) was added to the production medium at a speed of 1 mL/min. The culture was carried out while keeping the culture solution temperature at 37°C and controlling the pH constant at 6.9. In addition, the dissolved oxygen concentration in the culture solution was also maintained at 20% of the dissolved oxygen saturation concentration, and the culture was continued for 20 hours. Thereafter, the expression of the artificial fibroin was induced by adding 1 M isopropyl-β-thiogalactopyranoside (IPTG) to the culture solution to a final concentration of 1 mM. When 20 hours had elapsed after the addition of IPTG, the culture solution was centrifuged, and bacterial cells were collected. SDS-PAGE was conducted using the bacterial cells prepared from the culture solution obtained before the addition of IPTG and after the addition of IPTG. The expression of the target artificial fibroin that depended on the addition of IPTG was confirmed by emergence of a band having the size of the target artificial fibroin.

### (Purification of Polypeptide)

Bacterial cells that were collected 20 hours after the addition of IPTG were washed with a 20 mM Tris-HCl buffer (pH 7.4). The washed bacterial cells were suspended in a 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the precipitate reached a high level of purity. The washed precipitate was suspended in an 8 M guanidine buffer (8 M guanidine hydrochloride, 10 mM sodium dihydrogen phosphate, 20 mM NaCl, 1 mM Tris-HCl, pH 7.0) such that the precipitate reached a concentration of 100 mg/mL, and then, the precipitate was dissolved by being stirred with a stirrer at 60°C for 30 minutes. After the precipitate was dissolved, the obtained solution was dialyzed with water using a dialysis tube (cellulose tube 36/32 manufactured by Sanko Junyaku Co., Ltd.).

A white aggregated polypeptide obtained after dialysis was collected by centrifugation, and moisture was removed with a freeze dryer and a freeze-dried powder was collected, thereby obtaining a powdery artificial fibroin (PRT2882). It was confirmed that intermolecular disulfide bond was formed in the artificial fibroin into which a cysteine residue was inserted. It was confirmed that the artificial fibroin contained 13.8 parts by mass of a dimer, 2.1 parts by mass of a trimer, and 0.8 parts by mass of a tetramer, relative to 100 parts by mass of a monomer. Formation of a disulfide bond was measured by SDS-PAGE.

This artificial fibroin (PRT2882) was used as a protein CTR.

### <Production of Protein (Protein A) Having GFIL Sequences at Both Terminals>

Powdered artificial fibroin (PRT3046) was obtained in the same manner as for the powdered artificial fibroin (PRT2882), except that the amino acid sequence represented by SEQ ID NO: 10 (PRT3046) was used in place of the amino acid sequence shown in SEQ ID NO: 9 (PRT2882).

This artificial fibroin (PRT3046) was used as protein A.

### <Production of Protein B>

Powdered artificial fibroin (PRT3173) was obtained in the same manner as for the powdered artificial fibroin (PRT2882), except that the amino acid sequence represented by SEQ ID NO: 11 (PRT3173) was used in place of the amino acid sequence shown in SEQ ID NO: 9 (PRT2882).

This artificial fibroin (PRT3173) was used as protein B.

### <Production of Protein C>

Powdered artificial fibroin (PRT3174) was obtained in the same manner as for the powdered artificial fibroin (PRT2882), except that the amino acid sequence represented by SEQ ID NO: 12 (PRT3174) was used in place of the amino acid sequence shown in SEQ ID NO: 9 (PRT2882).

This artificial fibroin (PRT3174) was used as protein C.

### <Production of Protein D>

Powdered artificial fibroin (PRT3175) was obtained in the same manner as for the powdered artificial fibroin (PRT2882), except that the amino acid sequence represented by SEQ ID NO: 13 (PRT3175) was used in place of the amino acid sequence shown in SEQ ID NO: 9 (PRT2882).

This artificial fibroin (PRT3175) was used as protein D.

### <Production of Protein E>

Powdered artificial fibroin (PRT3176) was obtained in the same manner as for the powdered artificial fibroin (PRT2882), except that the amino acid sequence represented by SEQ ID NO: 14 (PRT3176) was used in place of the amino acid sequence shown in SEQ ID NO: 9 (PRT2882).

This artificial fibroin (PRT3176) was used as protein E.

### <Production of Protein F>

Powdered artificial fibroin (PRT3177) was obtained in the same manner as for the powdered artificial fibroin (PRT2882), except that the amino acid sequence represented by SEQ ID NO: 15 (PRT3177) was used in place of the amino acid sequence shown in SEQ ID NO: 9 (PRT2882).

This artificial fibroin (PRT3177) was used as protein F.

### <Production of Protein G>

Powdered artificial fibroin (PRT3463) was obtained in the same manner as for the powdered artificial fibroin (PRT2882), except that the amino acid sequence represented by SEQ ID NO: 16 (PRT3463) was used in place of the amino acid sequence shown in SEQ ID NO: 9 (PRT2882).

This artificial fibroin (PRT3463) was used as protein F.

The protein CTR is a protein in which the reaction site closest to the N-terminus side is present within the main sequence and the reaction site closest to the C-terminus side is present at the position of 10 residues from the C-terminus of the main sequence.

The protein A is a protein in which the reaction site closest to the N-terminus side is present within the main sequence and the reaction site closest to the C-terminus side is present at the position of 10 residues from the C-terminus of the main sequence.

The protein B is a protein in which the reaction site closest to the N-terminus side is not present within the main sequence and the reaction site closest to the C-terminus side is present at the position of 10 residues from the C-terminus of the main sequence.

The protein C is a protein in which the reaction site closest to the N-terminus side is not present within the main sequence and the reaction site closest to the C-terminus side is present at the position of 3 residues from the C-terminus of the main sequence.

The protein D is a protein in which the reaction site closest to the N-terminus side is not present within the main sequence and the reaction site closest to the C-terminus side is present at the C-terminus of the hydrophobic tag.

The protein E is a protein in which the reaction site closest to the N-terminus side is present within the main sequence and the reaction site closest to the C-terminus side is present at the position of 3 residues from the C-terminus of the main sequence.

The protein F is a protein in which the reaction site closest to the N-terminus side is present within the main sequence and the reaction site closest to the C-terminus side is present at the C-terminus of the hydrophobic tag.

The protein G is a protein in which the reaction site closest to the N-terminus side is present at the N-terminus of the hydrophobic tag and the reaction site closest to the C-terminus side is present at the C-terminus of the hydrophobic tag.

### [Method for Measuring Protein Production Amount]

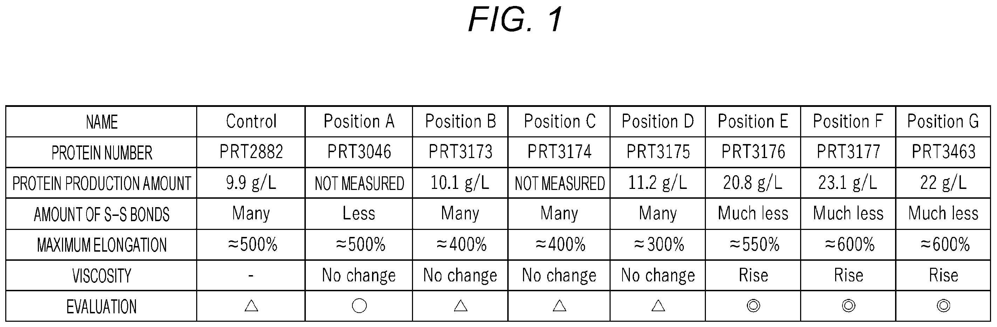

The purified product was freeze-dried to obtain a dry powder of the target protein. The weight of the powder was divided by the weight of the culture medium at the end of the culture to calculate the amount of powder acquired per 1 L of the culture medium, and the production amount was compared. The measurement results are described in the column of "protein production amount" in Fig. 1. A larger value indicates higher productivity of the artificial protein of the present invention.

### [Method for Measuring Amount of S-S Binding Amount]

The amount of the S-S binding amount was measured by using gel permeation chromatography (GPC).

The dried protein powder was analyzed as an HFIP solution containing 0.1% by weight sodium trifluoroacetate (final concentration: 1 mM). A sample was dissolved (45°C, 1,500 rpm, 1 hour) using a heating and vibrating device (Front Lab MyBL-100S, manufactured by AS ONE Corporation). The obtained solution was caused to pass through a hydrophilic PTFE membrane filter having a pore size of 0.45 µm (trade name: Dismic 25HP45AN, manufactured by ADVANTEC CO.,LTD.), and remaining insoluble matters were removed.

The GPC used was an Agilent 1260 Infinity II liquid chromatography system (Agilent Technologies, Inc. equipped with a refractive index (RI) detector and a styrenedivinylbenzene copolymer column (4.6 mm I.D. × 150 mm) fitted with a 0.5 µm guard column filter (trade name: Shodex GPC HK-G, manufactured by Showa Denko K.K.). The mobile phase was monitored at a flow rate of 0.15 mL/min and 40°C using an Agilent 1260 Infinity II refractive index detector (RID) (Agilent Technologies, Inc.). In addition, as a Control powder, a solution in which an S-S bond was eliminated by performing a reduction treatment was prepared. The reduction treatment was performed by adding TCEP before heating and vibrating the sample solution (final concentration: 10 mM).

In the analysis of the data, the GPC peak of each protein sample not subjected to the reduction treatment was obtained based on the peak position of the Control sample subjected to the reduction treatment, and the peak at the same position as the peak position of the Control sample was determined as a monomer, and the peak appearing on the higher molecular weight side than the peak of the Contorl sample was determined as an S-S conjugate. The measurement results are described in the column of "S-S binding amount" in Fig. 1. A smaller S-S binding amount indicates higher reactivity of the protein toward the second segment.

### [Production of Block Copolymer (BCP)]

Each of the proteins CTR, A to F (2 g, 202 µmol (protein CTR), 186 µmol (proteins A to F)), about 10 kDa bis(monomalonate) A (2 g, 200 µmol), and reducing agent dithiothreitol (23.8 mg, 1.54 µmol) were suspended in DMSO (26 mL). Thereafter, while the mixture was heated to 85°C in an oil bath, the mixture was stirred for 120 minutes using a Hercules stirrer (Trade name: MODEL HERAXLES/16G, manufactured by Koike Precision Instruments Co., Ltd.) to obtain a brown clear solution. The solution was thinly applied to a metal plate, to which a release film (manufactured by Teijin Film Solution Co., Ltd., thickness: 38 µm) was attached, using a doctor blade (manufactured by Imoto machinery Co., Ltd., coating width: 80 mm, gap: 400 µm), dried at 60°C for 2 hours or more using an air constant temperature thermostat, and further the solvent was removed at 80°C for 15 hours using a vacuum oven to obtain a brown transparent film.

The structure of bis(monomalonate) A is as follows.

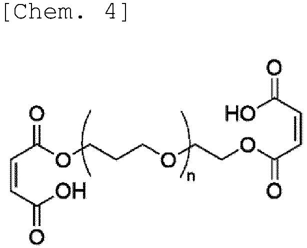

In addition, protein G (0.52 g, 47.2 µmol), PEG-I having the following structure of about 10 kDa (0.48 g, 200 µmol), and sodium cyanoborohydride (59.8 mg, 952 µmol) as a reducing agent were suspended in DMSO (17.3 mL). Thereafter, while the mixture was heated to 85°C in an oil bath, the mixture was stirred for 60 minutes using a Hercules stirrer (Trade name: MODEL HERAXLES/16G, manufactured by Koike Precision Instruments Co., Ltd.) to obtain a brown clear solution. The solution was thinly applied to a metal plate, to which a release film (manufactured by Teijin Film Solution Co., Ltd., thickness: 38 µm) was attached, using a doctor blade (manufactured by Imoto machinery Co., Ltd., coating width: 80 mm, gap: 400 µm). The applied solution was dried at 60°C for 2 hours or more using an air constant temperature thermostat, and the solvent was further removed at 80°C for 15 hours using a vacuum oven to obtain a transparent film.

The structure of PEG-I is as follows.

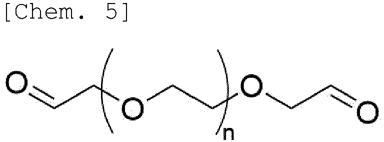

### [Method for Measuring Maximum Elongation]

The obtained film was punched into a dumbbell shape having a length of 105 mm and a width of 5 mm by a punching machine (SA-1008 manufactured by Tester Sangyo Co., Ltd.), and stored in an environment of a room temperature of 20°C and a humidity of 65% for 24 hours or more. The stored sample was subjected to a tensile test under the same environment using a tensile tester (AG-X plus50kN manufactured by Shimadzu Corporation) under the conditions of a load cell of 1 kN, a distance between chucks of 71.0 mm, and a tensile speed of 200 mm/min. The measurement results are described in the column of "maximum elongation" in Fig. 1. A larger value of the maximum elongation indicates that the first and second segments have reacted more sufficiently and that a sufficient amount of the block copolymer has been obtained.

### [Method for Determining Viscosity]

The viscosity was determined by the appearance at the time of film casting of the solution immediately after synthesis. Regarding the example evaluated as "Rise", compared to the example evaluated as "No change", it possessed viscosity and spinnability, and clearly showing an aspect different from the others.

A higher viscosity indicates that the first and second segments have reacted more sufficiently and that a sufficient amount of the block copolymer has been obtained.

From Fig. 1, in the production of the proteins D to G, an increase in the production amount was observed as compared with the production of the protein CTR. In Fig. 1, the protein CTR is described as "Control", and the proteins A to G are described as "Positions A to G", respectively.

From Fig. 1, as compared with the protein CTR, it is found that the viscosity does not change in the case of using the protein D in which the reaction site closest to the N-terminus side is moved into the hydrophobic tag, but the viscosity increases in the proteins F to G in which the reaction site closest to the C-terminus side is moved to the C-terminus of the hydrophobic tag or within 9 residues from the C-terminus of the main sequence. In general, since the viscosity increases as the reaction proceeds, it can be said that among the present molecular candidates, the reactivity is improved by setting the reaction site closest to the N-terminus side within the main sequence, and moving the reaction site closest to the C-terminus side to the C-terminus of the hydrophobic tag or within 9 residues from the C-terminus of the main sequence.

In addition, in Fig. 1, when the proteins CTR, E, and F are compared with each other, it is found that the maximum elongation of the film to be obtained increases when the reaction site closest to the N-terminus side is present within the main sequence, and the reaction site closest to the C-terminus side is present within the C-terminus of the hydrophobic tag on the C-terminus side, or is present within 9 residues from the C-terminus of the main sequence. In addition, it is found that the maximum elongation of the film to be obtained tends to increase as the reaction site closest to the N-terminus side is present within the main sequence and the position of the reaction site closest to the C-terminus side is brought closer to the C-terminus in the amino acid sequence having the main sequence and the hydrophobic tag. On the other hand, in the proteins B to D in which the reaction site closest to the N-terminus side is present in the hydrophobic tag, it is found that the maximum elongation is small.

Furthermore, from Fig. 1, it is found that proteins B to D, in which the reaction site closest to the N-terminus side was moved into the hydrophobic tag, have more S-S bonds than protein A, and proteins E and F, in which the reaction site closest to the C-terminus side was moved into the hydrophobic tag on the C-terminus side or within 9 residues from the C-terminus of the main sequence, have fewer S-S bonds than protein A.

From the above, it is found that a block copolymer using a protein that has a main sequence and hydrophobic tags at the N-terminus and the C-terminus of the main sequence, has at least two reaction sites that react with the compound having the second segment and a reactive group, in which the reaction site closest to the N-terminus side is present within the main sequence or within the hydrophobic tag on the N-terminus side, and the reaction site closest to the C-terminus side is present within the hydrophobic tag on the C-terminus side or is present within 9 residues from the C-terminus of the main sequence, exhibits superior productivity.

### (Example 2: Study on Block Copolymer (BCP) Using Protein G)

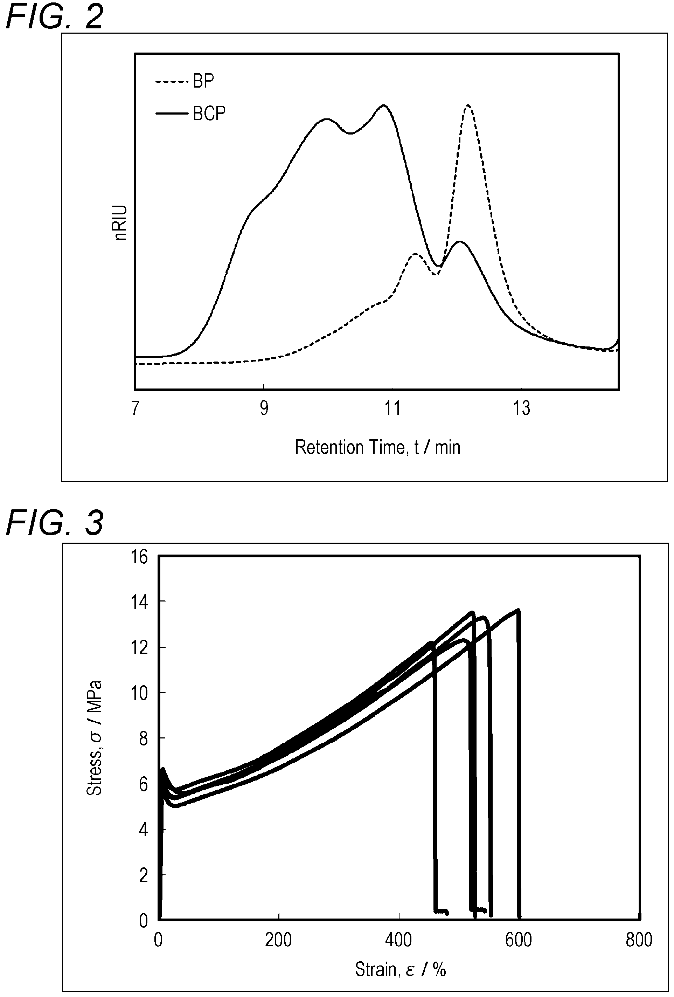

The film containing the block copolymer obtained using the above-described protein G was finely cut, dissolved in a TFA salt-containing HFIP together with a reducing agent TCEP, and subjected to GPC. As a result, the result of Fig. 2 was obtained. A broken line is a peak of protein G (PRT3463), and a solid line is a peak of BCP obtained.

In addition, BCP using bis(monomalonate) A was produced by the same method as in the production of the block copolymer obtained using the above-described protein G except that bis(monomalonate) A was used in place of PEG-I and dithiothreitol was used as a reducing agent. In the BCP, the peak of the raw material BP is considerably small, and a large peak appears in a high-molecular-weight part that is not observed in BP. This suggests that the PRT3463 and PEG-I react with each other to produce BCP.

The obtained film was subjected to a tensile test by the same method as other BCPs. The result is shown in Fig. 3. As in the case of BCP using bis(monomalonate) A, a film that was soft and highly stretchable could be prepared.

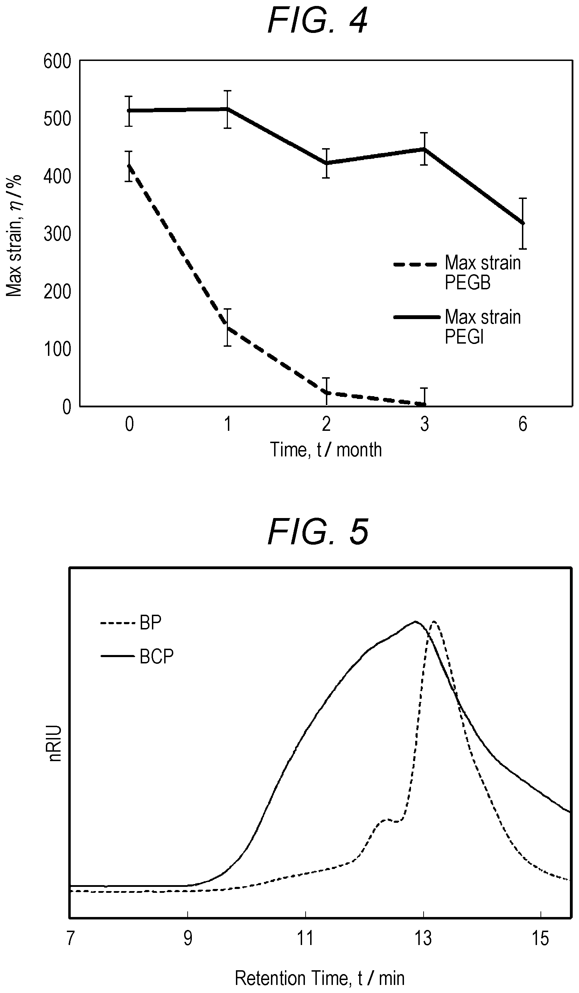

The obtained film was subjected to a jungle test under the conditions of a temperature of 60°C and a humidity of 60% to examine deterioration characteristics of the film. The result is shown in Fig. 4. The dotted line is the BCP result of bis(monomalonate) A, and the solid line is the BCP result of PEG-I.

It was found that deterioration of elongation was more suppressed in BCP using PEG-I than in BCP using bis(monomalonate) A (a film produced using the above-described protein F). In addition, changes in physical properties were shown, suggesting that the reaction via lysine rather than cysteine proceeded.

### (Example 3: Synthesis of BCP Using PCB)

A protein (2.25 g, 209 µmol) having an amino acid sequence represented by SEQ ID NO: 15 (PRT3177) and a reducing agent dithiothreitol (193.1 mg, 1.25 mmol) were suspended in DMSO (21.8 mL). Thereafter, the mixture was reduced for 30 minutes using a Hercules stirrer (Trade name: MODEL HERAXLES/16G, manufactured by Koike Precision Instruments Co., Ltd.) while being heated to 85°C in an oil bath. Thereafter, a PCB of about 3kDa (3.75 g, 375 µmol) was put in and stirred again in an oil bath at 85°C for 30 minutes to obtain a brown clear solution.

The structure of the PCB is as follows.

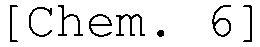

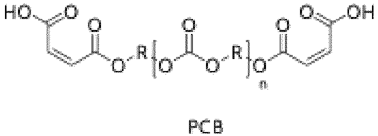

The solution was thinly applied to a metal plate, to which a release film (manufactured by Teijin Film Solution Co., Ltd., thickness: 38 µm) was attached, using a doctor blade (manufactured by Imoto machinery Co., Ltd., coating width: 80 mm, gap: 400 µm). The applied solution was dried at 60°C for 2 hours or more using an air constant temperature thermostat, and the solvent was further removed at 80°C for 15 hours using a vacuum oven to obtain a transparent film.

The obtained film was finely cut, dissolved in TFA salt-containing HFIP together with a reducing agent TCEP, and subjected to GPC. As a result, the result of Fig. 5 was obtained. A broken line is a peak of the PRT3177, and a solid line is a peak of the obtained BCP.

The peak of the BCP largely appeared in the high-molecular-weight part which was not observed in the PRT3177. This suggested that the PRT3177 reacts with the PCB to make the BCP.

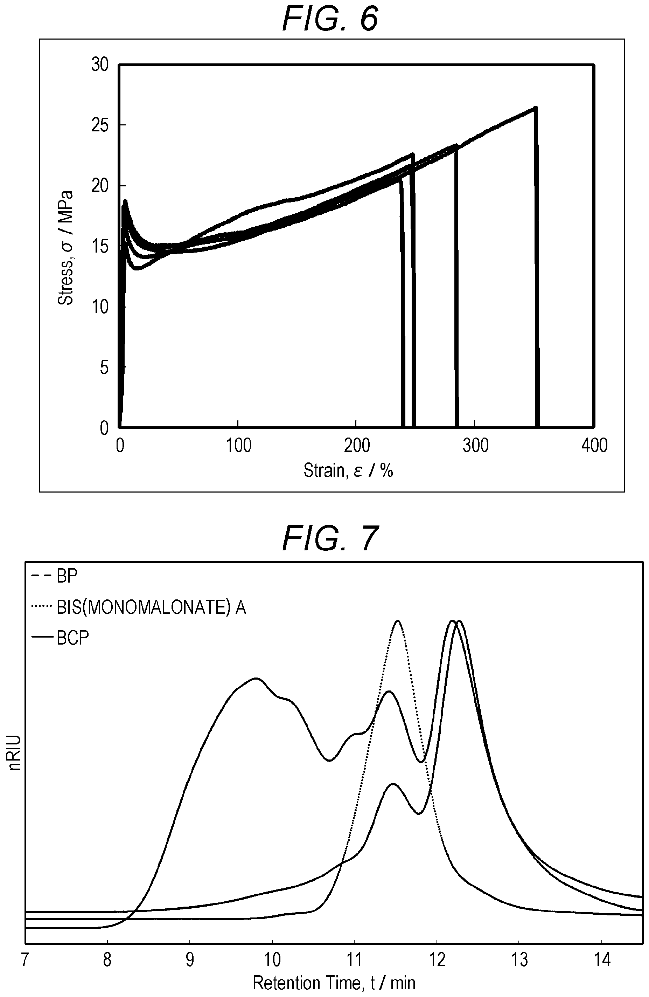

The obtained film was subjected to a tensile test by the same method as other BCPs. The result is shown in Fig. 6. It was confirmed that the prepared film stretched by about 300%.

In addition, the block copolymer using PCB has an advantage that the tensile yield strength is improved and the water swelling rate is reduced (hydrolyzability is reduced).

### (Example 4: Synthesis of BCP by Mechanochemical Method)

49 g of a dried protein (PRT3177, 10,763 g/mol) having the amino acid sequence represented by SEQ ID NO: 15 (PRT3177), 51 g of bis(monomalonate) A (11,000 g/mol) (molar ratio: 1:1), and 0.49 g of a reducing agent DTT (BP ratio: 1% by weight) were thoroughly mixed. DMSO was put thereto in an amount of 11 mL (solid content ratio: 12.1% by weight), and the mixture was thoroughly mixed beforehand.

The mixture was put little by little at a rate of 288 g/h in a twin-screw extruding/kneading machine (manufactured by TECHNOVEL CORPORATION) at 60°C and an extrusion rate of 273 g/h, and the substance that came out was subjected to GPC measurement by the same instrument and method as those of other BCPs.

As a result, the result of Fig. 7 was obtained. A broken line is a peak of the protein F (PRT3463), and a solid line is a peak of BCP obtained.

As a result of GPC measurement, a large GPC peak was observed at a position on the higher molecular weight side than the protein (BP) and PEG of the raw materials, and it was suggested that BCP synthesis proceeded even when PRT3177 was used.

## Claims

1. A block copolymer comprising a first segment including a protein structure, and one or a plurality of second segments including a molecular group having a function of plasticizing the protein structure and bound to the first segment, wherein
the protein structure has a main sequence and a hydrophobic tag bound to an N-terminus and a C-terminus of the main sequence, and has at least two reaction sites that react with a compound having the second segment and a reactive group,
among the reaction sites, the reaction site closest to the N-terminus side is present within the hydrophobic tag on the N-terminus side, is NH₂ that is the N-terminus of the protein structure, or is present within the main sequence, and
among the reaction sites, the reaction site closest to the C-terminus side is present within the hydrophobic tag on the C-terminus side or is present within 9 residues from the C-terminus of the main sequence.

2. The block copolymer according to claim 1, wherein the reaction site closest to the C-terminus side among the reaction sites is present at the C-terminus of the hydrophobic tag on the C-terminus side, or is present within 9 residues from the C-terminus of the main sequence.

3. The block copolymer according to claim 1 or 2, wherein the reaction site closest to the N-terminus side among the reaction sites is present at the N-terminus of the hydrophobic tag on the N-terminus side, or is present within the main sequence.

4. The block copolymer according to any one of claims 1 to 3, wherein, among the reaction sites, the reaction site closest to the N-terminus side, which is present within the main sequence, and the reaction site closest to the C-terminus side, which is present within the hydrophobic tag on the C-terminus side or is present within 9 residues from the C-terminus of the main sequence, are thiol groups.

5. The block copolymer according to claim 4, wherein the compound has a thiol-reactive group that reacts with the thiol.

6. The block copolymer according to any one of claims 1 to 3, wherein, among the reaction sites, the reaction site closest to the N-terminus side, which is present within the hydrophobic tag on the N-terminus side, and the reaction site closest to the C-terminus side, which is present within the hydrophobic tag on the C-terminus side or is present within 9 residues from the C-terminus of the main sequence, are amino groups.

7. The block copolymer according to claim 6, wherein the compound has an amino-reactive group that reacts with the amine.

8. The block copolymer according to any one of claims 1 to 7, wherein the hydrophobic tag is GFIL or two or more repeats of GFIL.

9. The block copolymer according to any one of claims 1 to 8, wherein a molecular weight of the protein structure is 1 to 300 kDa.

10. A method for producing a block copolymer, the method comprising binding a compound having a first segment including a protein structure, and a compound having a second segment including a molecular group having a function of plasticizing the protein structure, wherein
the protein structure has a main sequence and a hydrophobic tag bound to an N-terminus and a C-terminus of the main sequence, and has at least two reaction sites that react with a compound having the second segment and a reactive group,
among the reaction sites, the reaction site closest to the N-terminus side is present within the hydrophobic tag on the N-terminus side, is NH₂ that is the N-terminus of the protein structure, or is present within the main sequence, and
among the reaction sites, the reaction site closest to the C-terminus side is present within the hydrophobic tag on the C-terminus side or is present within 9 residues from the C-terminus of the main sequence.

11. The method for producing a block copolymer according to claim 10, comprising a step of binding the compound having the first segment and the compound having the second segment by a mechanochemical treatment.

12. The method for producing a block copolymer according to claim 10, comprising adding the compound having the first segment and the compound having the second segment, to a solvent, wherein the compound having the second segment is added in a non-solution state.

13. The method for producing a block copolymer according to claim 12, comprising adding a reducing agent, a compound having the first segment, and a compound having the second segment, to the solvent in this order.

14. An artificial protein comprising a main sequence and a hydrophobic tag bound to an N-terminus and a C-terminus of the main sequence, and the artificial protein including hydrophobic tags at the N-terminus and the C-terminus, wherein
the artificial protein contains at least two cysteine residues,
among the cysteine residues, a cysteine residue closest to the N-terminus side is present within the main sequence, and
among the cysteine residues, a cysteine residue closest to the C-terminus side is present within the hydrophobic tag on the C-terminus side or present within 9 residues from the C-terminus of the main sequence.

15. An artificial protein comprising a main sequence and a hydrophobic tag bound to an N-terminus and a C-terminus of the main sequence, and the artificial protein including hydrophobic tags at the N-terminus and the C-terminus, wherein
the artificial protein contains at least one lysine residue,
among the lysine residues, a lysine residue closest to the N-terminus side is present within the hydrophobic tag on the N-terminus side, or no lysine residue is present within the hydrophobic tag on the N-terminus side, and
among the lysine residues, a lysine residue closest to the C-terminus side is present within the hydrophobic tag on the C-terminus side or present within 9 residues from the C-terminus of the main sequence.

16. The artificial protein according to claim 14 or 15, wherein the hydrophobic tag is GFIL or two or more repeats of GFIL.

17. The artificial protein according to any one of claims 14 to 16, wherein a molecular weight is 1 to 300 kDa.
